# EUROPEAN PATENT APPLICATION

(11) **EP 3 677 594 A1**
(43) Date of publication of application: **08.07.2020**
(21) Application number: 19150500.7
(22) Date of filing: 07.01.2019
(51) Int. Cl.: C07K 14/24, C12P 13/22

(54) **METHOD FOR PRODUCING L-TRYPTOPHAN USING IMPROVED STRAINS OF THE ENTEROBACTERIACEAE FAMILY**

(71) Applicant: Evonik Operations GmbH, 45128 Essen (DE)
(72) Inventor: RIEPING, Mechthild, 33619 Bielefeld (DE); JERRENTRUP, Silke, 33607 Bielefeld (DE); DUSCH, Nicole, 33824 Werther (DE)
(74) Representative: Evonik Patent Association

(57) **Abstract**

The invention provides a method of producing L-tryptophan, the method comprising culturing a L-tryptophan producing microorganism belonging to the *Enterobacteriaceae* family in a fermentation medium; wherein the L-tryptophan producing microorganism has been modified by enhancing the expression level of the mdfA gene or by enhancing the expression level of an mdfA allele.

SEQUENCE LISTING

<110>  Evonik Degussa GmbH

<120>  Method for producing L-tryptophan using improved strains of the
       Enterobacteriaceae family

<130>  201800223

<160>  25

<170>  PatentIn version 3.5

<210>  1
<211>  1233
<212>  DNA
<213>  Escherichia coli


<220>
<221>  CDS
<222>  (1)..(1233)
<223>  Coding region mdfA

<400>  1
atg caa aat aaa tta gct tcc ggt gcc agg ctt gga cgt cag gcg tta        48
Met Gln Asn Lys Leu Ala Ser Gly Ala Arg Leu Gly Arg Gln Ala Leu
1               5                   10                  15

ctt ttc cct ctc tgt ctg gtg ctt tac gaa ttt tca acc tat atc ggc        96
Leu Phe Pro Leu Cys Leu Val Leu Tyr Glu Phe Ser Thr Tyr Ile Gly
            20                  25                  30

aac gat atg att caa ccc ggt atg ttg gcc gtg gtg gaa caa tat cag       144
Asn Asp Met Ile Gln Pro Gly Met Leu Ala Val Val Glu Gln Tyr Gln
        35                  40                  45

gcg ggc att gat tgg gtt cct act tcg atg acc gcg tat ctg gcg ggc       192
Ala Gly Ile Asp Trp Val Pro Thr Ser Met Thr Ala Tyr Leu Ala Gly
    50                  55                  60

ggg atg ttt tta caa tgg ctg ctg ggg ccg ctg tcg gat cgt att ggt       240
Gly Met Phe Leu Gln Trp Leu Leu Gly Pro Leu Ser Asp Arg Ile Gly
65                  70                  75                  80

cgc cgt ccg gtg atg ctg gcg gga gtg gtg tgg ttt atc gtc acc tgt       288
Arg Arg Pro Val Met Leu Ala Gly Val Val Trp Phe Ile Val Thr Cys
                85                  90                  95

ctg gca ata ttg ctg gcg caa aac att gaa caa ttc acc ctg ttg cgc       336
Leu Ala Ile Leu Leu Ala Gln Asn Ile Glu Gln Phe Thr Leu Leu Arg
            100                 105                 110

ttc ttg cag ggc ata agc ctc tgt ttc att ggc gct gtg gga tac gcc       384
Phe Leu Gln Gly Ile Ser Leu Cys Phe Ile Gly Ala Val Gly Tyr Ala
        115                 120                 125

gca att cag gaa tcc ttc gaa gag gcg gtt tgt atc aag atc acc gcg       432
Ala Ile Gln Glu Ser Phe Glu Glu Ala Val Cys Ile Lys Ile Thr Ala
    130                 135                 140

ctg atg gcg aac gtg gcg ctg att gct ccg cta ctt ggt ccg ctg gtg       480
Leu Met Ala Asn Val Ala Leu Ile Ala Pro Leu Leu Gly Pro Leu Val

145                 150                 155                 160

ggc gcg gcg tgg atc cat gtg ctg ccc tgg gag ggg atg ttt gtt ttg         528
Gly Ala Ala Trp Ile His Val Leu Pro Trp Glu Gly Met Phe Val Leu
                165                 170                 175

ttt gcc gca ttg gca gcg atc tcc ttt ttc ggt ctg caa cga gcc atg         576
Phe Ala Ala Leu Ala Ala Ile Ser Phe Phe Gly Leu Gln Arg Ala Met
            180                 185                 190

cct gaa acc gcc acg cgt ata ggc gag aaa ctg tca ctg aaa gaa ctc         624
Pro Glu Thr Ala Thr Arg Ile Gly Glu Lys Leu Ser Leu Lys Glu Leu
        195                 200                 205

ggt cgt gac tat aag ctg gtg ctg aag aac ggc cgc ttt gtg gcg ggg         672
Gly Arg Asp Tyr Lys Leu Val Leu Lys Asn Gly Arg Phe Val Ala Gly
    210                 215                 220

gcg ctg gcg ctg gga ttc gtt agt ctg ccg ttg ctg gcg tgg atc gcc         720
Ala Leu Ala Leu Gly Phe Val Ser Leu Pro Leu Leu Ala Trp Ile Ala
225                 230                 235                 240

cag tcg ccg att atc atc att acc ggc gag cag ttg agc agc tat gaa         768
Gln Ser Pro Ile Ile Ile Ile Thr Gly Glu Gln Leu Ser Ser Tyr Glu
                245                 250                 255

tat ggc ttg ctg caa gtg cct att ttc ggg gcg tta att gcg ggt aac         816
Tyr Gly Leu Leu Gln Val Pro Ile Phe Gly Ala Leu Ile Ala Gly Asn
            260                 265                 270

ttg ctg tta gcg cgt ctg acc tcg cgc cgc acc gta cgt tcg ctg att         864
Leu Leu Leu Ala Arg Leu Thr Ser Arg Arg Thr Val Arg Ser Leu Ile
        275                 280                 285

att atg ggc ggc tgg ccg att atg att ggt cta ttg gtc gct gct gcg         912
Ile Met Gly Gly Trp Pro Ile Met Ile Gly Leu Leu Val Ala Ala Ala
    290                 295                 300

gca acg gtt atc tca tcg cac gcg tat tta tgg atg act gcc ggg tta         960
Ala Thr Val Ile Ser Ser His Ala Tyr Leu Trp Met Thr Ala Gly Leu
305                 310                 315                 320

agt att tat gct ttc ggt att ggt ctg gcg aat gcg gga ctg gtg cga        1008
Ser Ile Tyr Ala Phe Gly Ile Gly Leu Ala Asn Ala Gly Leu Val Arg
                325                 330                 335

tta acc ctg ttt gcc agc gat atg agt aaa ggt acg gtt tct gcc gcg        1056
Leu Thr Leu Phe Ala Ser Asp Met Ser Lys Gly Thr Val Ser Ala Ala
            340                 345                 350

atg gga atg ctg caa atg ctg atc ttt acc gtt ggt att gaa atc agc        1104
Met Gly Met Leu Gln Met Leu Ile Phe Thr Val Gly Ile Glu Ile Ser
        355                 360                 365

aaa cat gcc tgg ctg aac ggg ggc aac gga ctg ttt aat ctc ttc aac        1152
Lys His Ala Trp Leu Asn Gly Gly Asn Gly Leu Phe Asn Leu Phe Asn
    370                 375                 380

ctt gtc aac gga att ttg tgg ctg tcg ctg atg gtt atc ttt tta aaa        1200
Leu Val Asn Gly Ile Leu Trp Leu Ser Leu Met Val Ile Phe Leu Lys
385                 390                 395                 400

gat aaa cag atg gga aat tct cac gaa ggg taa                            1233

Asp Lys Gln Met Gly Asn Ser His Glu Gly
                405                 410

<210>  2
<211>  410
<212>  PRT
<213>  Escherichia coli

<400>  2

Met Gln Asn Lys Leu Ala Ser Gly Ala Arg Leu Gly Arg Gln Ala Leu
1               5                   10                  15

Leu Phe Pro Leu Cys Leu Val Leu Tyr Glu Phe Ser Thr Tyr Ile Gly
            20                  25                  30

Asn Asp Met Ile Gln Pro Gly Met Leu Ala Val Val Glu Gln Tyr Gln
        35                  40                  45

Ala Gly Ile Asp Trp Val Pro Thr Ser Met Thr Ala Tyr Leu Ala Gly
    50                  55                  60

Gly Met Phe Leu Gln Trp Leu Leu Gly Pro Leu Ser Asp Arg Ile Gly
65                  70                  75                  80

Arg Arg Pro Val Met Leu Ala Gly Val Val Trp Phe Ile Val Thr Cys
                85                  90                  95

Leu Ala Ile Leu Leu Ala Gln Asn Ile Glu Gln Phe Thr Leu Leu Arg
            100                 105                 110

Phe Leu Gln Gly Ile Ser Leu Cys Phe Ile Gly Ala Val Gly Tyr Ala
        115                 120                 125

Ala Ile Gln Glu Ser Phe Glu Glu Ala Val Cys Ile Lys Ile Thr Ala
    130                 135                 140

Leu Met Ala Asn Val Ala Leu Ile Ala Pro Leu Leu Gly Pro Leu Val
145                 150                 155                 160

Gly Ala Ala Trp Ile His Val Leu Pro Trp Glu Gly Met Phe Val Leu
                165                 170                 175

Phe Ala Ala Leu Ala Ala Ile Ser Phe Phe Gly Leu Gln Arg Ala Met
            180                 185                 190

Pro Glu Thr Ala Thr Arg Ile Gly Glu Lys Leu Ser Leu Lys Glu Leu
        195                 200                 205

Gly Arg Asp Tyr Lys Leu Val Leu Lys Asn Gly Arg Phe Val Ala Gly
    210                 215                 220

Ala Leu Ala Leu Gly Phe Val Ser Leu Pro Leu Leu Ala Trp Ile Ala
225                 230                 235                 240

Gln Ser Pro Ile Ile Ile Ile Thr Gly Glu Gln Leu Ser Ser Tyr Glu
                245                 250                 255

Tyr Gly Leu Leu Gln Val Pro Ile Phe Gly Ala Leu Ile Ala Gly Asn
            260                 265                 270

Leu Leu Leu Ala Arg Leu Thr Ser Arg Arg Thr Val Arg Ser Leu Ile
        275                 280                 285

Ile Met Gly Gly Trp Pro Ile Met Ile Gly Leu Leu Val Ala Ala Ala
    290                 295                 300

Ala Thr Val Ile Ser Ser His Ala Tyr Leu Trp Met Thr Ala Gly Leu
305                 310                 315                 320

Ser Ile Tyr Ala Phe Gly Ile Gly Leu Ala Asn Ala Gly Leu Val Arg
                325                 330                 335

Leu Thr Leu Phe Ala Ser Asp Met Ser Lys Gly Thr Val Ser Ala Ala
            340                 345                 350

Met Gly Met Leu Gln Met Leu Ile Phe Thr Val Gly Ile Glu Ile Ser
        355                 360                 365

Lys His Ala Trp Leu Asn Gly Gly Asn Gly Leu Phe Asn Leu Phe Asn
    370                 375                 380

Leu Val Asn Gly Ile Leu Trp Leu Ser Leu Met Val Ile Phe Leu Lys
385                 390                 395                 400

Asp Lys Gln Met Gly Asn Ser His Glu Gly
                405                 410

<210>  3
<211>  35
<212>  DNA
<213>  Escherichia coli

<220>
<221>  misc_feature
<222>  (1)..(35)
<223>  Primer aroP-up1

<400>  3
gatctgagct ctagacctgg cgacgaagca acaag                                35

<210>  4
<211>  37
<212>  DNA
<213>  Escherichia coli

<220>
<221>  misc_feature
<222>  (1)..(37)
<223>  Primer aroP-up6

<400>  4
gcgttggtgt aatcgcgaac ctcgtgcggt ggttgtt                              37

<210>  5
<211>  31
<212>  DNA
<213>  Escherichia coli

<220>
<221>  misc_feature
<222>  (1)..(31)
<223>  Primer aroP-down2

<400>  5
gatctatcta gactgttacg cgcattgcag g                                    31

<210>  6
<211>  41
<212>  DNA
<213>  Escherichia coli

<220>
<221>  misc_feature
<222>  (1)..(41)
<223>  Primer aroP-down3

<400>  6
accgcacgag gttcgcgatt acaccaacgc cccgtaaatc g                         41

<210>  7
<211>  21
<212>  DNA
<213>  miscellaneous

<220>
<221>  misc_feature
<222>  (1)..(21)
<223>  Primer pKO3-L

<400>  7
agggcagggt cgttaaatag c                                               21

<210> 8
<211> 21
<212> DNA
<213> miscellaneous

<220>
<221> misc_feature
<222> (1)..(21)
<223> Primer pKO3-R

<400> 8
ttaatgcgcc gctacagggc g 21

<210> 9
<211> 1568
<212> DNA
<213> Escherichia coli

<220>
<221> misc_feature
<222> (1)..(1568)
<223> DaroP; construct for deletion of aroP

<400> 9
agggcagggt cgttaaatag ccgcttatgt ctattgctgg tctcggtacc cggggatcgc 60

ggccgcggac cggatcctct agacctggcg acgaagcaac aagcccttcg cttcgagacg 120

ttgaatcgcc tcacgcaagg agggacggga gacgtcaaac tgttttgcca gttcgcgttc 180

cggtgggagt ttttcgcccg ggcggagagt gccttcgagg atcaaaaact ccagttgctg 240

ctcaatcaca tcggagagtt ttggttggcg gattttgctg taggccatga gttcctgtct 300

taagccactt gccgaagtca attggtctta ccaatttcat gtctgtgacg ctaaagtaac 360

aaagtattca ccttatgtcc atacaggttt tgattgaaat catgaaactg tgcacatttt 420

aacaacttga catatataac gtttcaaagt tgtaactatg cacaaatgta gactttacgt 480

aggaaaggag ttttttaacg attattaaac tataaaaatc cgaattgaac cgattcactt 540

accaattttg tgatttttaa ttcaattaaa acgaatttaa attcattcta catattgaga 600

ggggttgagg ctgagcttta caaacggttt ctttttaagc aactcatctt caaccatgca 660

taaagcgggt gcattcgctg ccgcatacca ttattcttga tctgacggaa gtctttttgt 720

aacaattcaa acttctttga tgtaaacaaa ttaatacaac aaacggaatt gcaaacttac 780

acacgcatca ctgcgtagat caaaaaaaca accaccgcac gaggttcgcg attacaccaa 840

cgccccgtaa atcgtcagta gtgcaatcac caccacgacc acgaacgagg ttttcttcgc 900

cattgaaacc gctgccttcg gcgtctccac cttatcgaca tgcggttcac gcgccagaga 960

gaactgcgcc agacgcgtta acacctgata ctgcgaagta tggaaatcac ccagcgaagc 1020

aaaccaggcc ggtaacgctt tctcaccatg accgatcaag gcatatacca cacccgcaag 1080

acgaaccggc acccaatcca gtacatgaag cacggcatca atgccggact gtaaacgatg     1140

atgcggcgtc tgatatcgcg ccagccagta ttgccatgca cgcaaaaacg cataccccat    1200

cagcgtaacg ggtccccagg ttccccccac aatcagccag aacagcggtg caagataaaa    1260

acgaaagtta atccacagca atgcattttg cagctcacgc aaatactcac gttcgtcgca    1320

gcctgccggg acgccgtgaa tcatggtgag ttcgccagcc atcgtggcac gggcatggct    1380

atcattacgt gaagcagctg tcagataagc atgataatga agacgaactt tacctgcgcc    1440

aatacacagc aaaccaatca gcagccacac cagtagcgtg ggaacgttga acaatactcc    1500

ctgcaatgcg cgtaacagtc tagagtcgac cggtggcgaa tgggacgcgc cctgtagcgg    1560

cgcattaa                                                              1568

<210>  10
<211>  23
<212>  DNA
<213>  Escherichia coli

<220>
<221>  misc_feature
<222>  (1)..(23)
<223>  Primer mtr-del-Xba-1

<400>  10
ttgagaaccg cgagcgtcgt ctg                                             23

<210>  11
<211>  32
<212>  DNA
<213>  Escherichia coli

<220>
<221>  misc_feature
<222>  (1)..(32)
<223>  Primer mtr-del-Xba-2

<400>  11
tctagattcg cgagggcttc tctccagtga aa                                   32

<210>  12
<211>  39
<212>  DNA
<213>  Escherichia coli

<220>
<221>  misc_feature
<222>  (1)..(39)
<223>  Primer mtr-del-Xba-3

<400>  12
aagccctcgc gaatctagag taatcagcgg tgccttatc                            39

<210>  13
<211>  22
<212>  DNA
<213>  Escherichia coli

<220>
<221>  misc_feature
<222>  (1)..(22)
<223>  Primer mtr-del-Xba-4

<400>  13
gctggcagaa caccacaata tg                                                22

<210>  14
<211>  2183
<212>  DNA
<213>  Escherichia coli

<220>
<221>  misc_feature
<222>  (1)..(2183)
<223>  Dmtr; construct for deletion of mtr

<400>  14
agggcagggt cgttaaatag ccgcttatgt ctattgctgg tctcggtacc cggggatcgc      60

ggccgccagt gtgatggata tctgcagaat tcgcccttgc tggcagaaca ccacaatatg     120

actggcattg ccagtgctgc cacgtcggta ttgatcggct atctggcggc gaataccacc     180

acgctgcatc tggggtctgg cggcgtgatg ttgcctaacc actcaccgtt ggtcattgca     240

gaacagttcg gcacgcttaa tacactctat ccggggcgaa tcgatttggg gctgggtcgt     300

gctccgggta gtgaccaacg gacaatgatg gcgctacgtc gtcatatgag cggcgatatt     360

gataatttcc cccgcgatgt ggcggagctg gtggactggt ttgacgcccg cgatcccaat     420

ccgcatgtgc gcccggtacc aggctatggc gagaaaatcc ccgtgtggtt gttaggctcc     480

agcctttaca gcgcgcaact ggcggcgcag cttggtctgc cgtttgcgtt tgcctcacac     540

ttcgcgccgg atatgctgtt ccaggcgctg catctttatc gcagcaactt caaaccgtca     600

gcacggctgg aaaaaccata cgcgatggtg tgcatcaata ttatcgccgc cgacagcaac     660

cgcgacgctg aatttctgtt tacctcaatg cagcaagcct ttgtgaagct gcgccgtggc     720

gaaaccgggc aactgccgcc gccgattcaa aatatggatc agttctggtc accgtctgag     780

cagtatggcg tgcagcaggc gctgagtatg tcgttggtag gtgataaagc gaaagtgcgt     840

catggcttgc agtcgatcct gcgcgaaacc gacgccgatg agattatggt caacgggcag     900

attttcgacc accaggcgcg gctgcattcg tttgagctgg cgatggatgt taaggaagag     960

ttgttgggat agtgtgtctt aacgcgggaa gccttatccg agctggcaac gctgtcctac    1020

atagacctga taagcgaagc gcatcaggca ttgtgtaggc agcagaaatg tcggataagg 1080

caccgctgat tactctagat tcgcgagggc ttctctccag tgaaaaatag tgcgactgcg 1140

ttgttatgca ttgcactgta ccagtacacg agtacaaaag acagaaaaaa agccccgatg 1200

gtaaaaatcg gggctgtata tattatttta cagattgtgt tcgctgttca gcgatgatta 1260

cgcatcacca ccgaaacgac gacgaccggt agaatcatca cgacgcggag cgcggccttc 1320

acgacgttcg ccgctaaaac gacgaccatc accacggcca ccttcacggc gttcaccgct 1380

gaagttacga ccgccttcac gacgttcgcc accgaaacca cgaccaccgc cacgacgctc 1440

accgccagta tgcggctgtg catcgcccag taactgcatg ttcatcggct tgttgagaat 1500

gcgagtgcgc gtaaagtgtt gcagcacttc acccggcata cctttcggca gttcgatggt 1560

ggagtgagaa gcaaacagct tgatgttacc aatgtaacgg ctgctgatgt cgccttcgtt 1620

agcaatcgca ccaacgatat gacgaacttc aacaccatca tcgcggccca cttcaatgcg 1680

gtacagctgc atatcgccaa catcacgacg ttcacgacgc ggacgatctt cacggtcacc 1740

acgcgggcca cggtcgttac gatcgcgcgg accacggtca tcacggtcac ggaattcacg 1800

tttcggacgc atcggcgcat ctggcggtac gatcagagta cgttcaccct gtgccatttt 1860

cagcagtgcc gcagccagag tttcgagatc cagctcttca ccttcagcag tcggctgaat 1920

tttgctcagc agtgcgcggt attgatccag atcgctgctt tccagctgct gctgtacttt 1980

agcggcgaat ttttccagac ggcgtttgcc tagcagttct gcgttcggca gttctacttc 2040

cggaatagtc agcttcatag tacgttcaat gttgcgcagc agacgacgct cgcggttctc 2100

aaaagggcga attccagcac actggcggcc gttactagag tcgaccggtg gcgaatggga 2160

cgcgccctgt agcggcgcat taa 2183

<210> 15
<211> 26
<212> DNA
<213> miscellaneous

<220>
<221> misc_feature
<222> (1)..(26)
<223> Primer cmr-for

<400> 15
cgtcgcgata caggcaagtc gttgag 26

<210> 16
<211> 26
<212> DNA
<213> miscellaneous

<220>

<221>  misc_feature
<222>  (1)..(26)
<223>  Primer cmr-rev

<400>  16
cctcgcgaca gattgacgac catcac                                          26


<210>  17
<211>  18
<212>  DNA
<213>  miscellaneous


<220>
<221>  misc_feature
<222>  (1)..(18)
<223>  Primer M13uni(-21)

<400>  17
tgtaaaacga cggccagt                                                   18


<210>  18
<211>  18
<212>  DNA
<213>  miscellaneous


<220>
<221>  misc_feature
<222>  (1)..(18)
<223>  Primer M13rev(-29)

<400>  18
caggaaacag ctatgacc                                                   18


<210>  19
<211>  1562
<212>  DNA
<213>  Escherichia coli


<220>
<221>  misc_feature
<222>  (1)..(1562)
<223>  mdfA CDS with promoter and terminator

<400>  19
cgtcgcgata caggcaagtc gttgagaata aagtgcaggt taaactgggt aaagcggcat      60

cgtcttattt ccctcaagcg gcctgtttac ggtgggtgat tgtaacgggc ataggttaaa     120

taaaacttaa agaaagcgta gctatactcg taataatgta agaatgtgct taaccgtggt     180

ttcagctaca aaattcgctt tctcgttagc tgcgctttta ttaaactctg cgcgattatt     240

attggcgaag aaattgcatg caaaataaat tagcttccgg tgccaggctt ggacgtcagg     300

cgttactttt ccctctctgt ctggtgcttt acgaattttc aacctatatc ggcaacgata     360

tgattcaacc cggtatgttg gccgtggtgg aacaatatca ggcgggcatt gattgggttc     420

ctacttcgat gaccgcgtat ctggcgggcg ggatgttttt acaatggctg ctggggccgc 480

tgtcggatcg tattggtcgc cgtccggtga tgctggcggg agtggtgtgg tttatcgtca 540

cctgtctggc aatattgctg gcgcaaaaca ttgaacaatt caccctgttg cgcttcttgc 600

agggcataag cctctgtttc attggcgctg tgggatacgc cgcaattcag gaatccttcg 660

aagaggcggt ttgtatcaag atcaccgcgc tgatggcgaa cgtggcgctg attgctccgc 720

tacttggtcc gctggtgggc gcggcgtgga tccatgtgct gccctgggag gggatgtttg 780

ttttgtttgc cgcattggca gcgatctcct ttttcggtct gcaacgagcc atgcctgaaa 840

ccgccacgcg tataggcgag aaactgtcac tgaaagaact cggtcgtgac tataagctgg 900

tgctgaagaa cggccgcttt gtggcggggg cgctggcgct gggattcgtt agtctgccgt 960

tgctggcgtg gatcgcccag tcgccgatta tcatcattac cggcgagcag ttgagcagct 1020

atgaatatgg cttgctgcaa gtgcctattt tcggggcgtt aattgcgggt aacttgctgt 1080

tagcgcgtct gacctcgcgc cgcaccgtac gttcgctgat tattatgggc ggctggccga 1140

ttatgattgg tctattggtc gctgctgcgg caacggttat ctcatcgcac gcgtatttat 1200

ggatgactgc cgggttaagt atttatgctt tcggtattgg tctggcgaat gcgggactgg 1260

tgcgattaac cctgtttgcc agcgatatga gtaaaggtac ggtttctgcc gcgatgggaa 1320

tgctgcaaat gctgatcttt accgttggta ttgaaatcag caaacatgcc tggctgaacg 1380

ggggcaacgg actgtttaat ctcttcaacc ttgtcaacgg aattttgtgg ctgtcgctga 1440

tggttatctt tttaaaagat aaacagatgg gaaattctca cgaagggtaa aaaaatgcct 1500

gactgctttg tgcgatcagg cattctcgaa ttaatggtga tggtcgtcaa tctgtcgcga 1560

gg 1562

<210> 20
<211> 3120
<212> DNA
<213> Escherichia coli

<220>
<221> misc_feature
<222> (1)..(3120)
<223> DaroP::mdfA; construct for integration of mdfA in delta aroP

<400> 20
agggcagggt cgttaaatag ccgcttatgt ctattgctgg tctcggtacc cggggatcgc 60

ggccgcggac cggatcctct agacctggcg acgaagcaac aagcccttcg cttcgagacg 120

ttgaatcgcc tcacgcaagg agggacggga gacgtcaaac tgttttgcca gttcgcgttc 180

cggtgggagt ttttcgcccg ggcggagagt gccttcgagg atcaaaaact ccagttgctg 240

ctcaatcaca tcggagagtt ttggttggcg gattttgctg taggccatga gttcctgtct 300

taagccactt gccgaagtca attggtctta ccaatttcat gtctgtgacg ctaaagtaac 360

aaagtattca ccttatgtcc atacaggttt tgattgaaat catgaaactg tgcacatttt 420

aacaacttga catatataac gtttcaaagt tgtaactatg cacaaatgta gactttacgt 480

aggaaaggag ttttttaacg attattaaac tataaaaatc cgaattgaac cgattcactt 540

accaattttg tgatttttaa ttcaattaaa acgaatttaa attcattcta catattgaga 600

ggggttgagg ctgagcttta caaacggttt ctttttaagc aactcatctt caaccatgca 660

taaagcgggt gcattcgctg ccgcatacca ttattcttga tctgacggaa gtctttttgt 720

aacaattcaa acttctttga tgtaaacaaa ttaatacaac aaacggaatt gcaaacttac 780

acacgcatca ctgcgtagat caaaaaaaca accaccgcac gaggttcgcg atacaggcaa 840

gtcgttgaga ataaagtgca ggttaaactg ggtaaagcgg catcgtctta tttccctcaa 900

gcggcctgtt tacggtgggt gattgtaacg ggcataggtt aaataaaact taaagaaagc 960

gtagctatac tcgtaataat gtaagaatgt gcttaaccgt ggtttcagct acaaaattcg 1020

ctttctcgtt agctgcgctt ttattaaact ctgcgcgatt attattggcg aagaaattgc 1080

atgcaaaata aattagcttc cggtgccagg cttggacgtc aggcgttact tttccctctc 1140

tgtctggtgc tttacgaatt ttcaacctat atcggcaacg atatgattca acccggtatg 1200

ttggccgtgg tggaacaata tcaggcgggc attgattggg ttcctacttc gatgaccgcg 1260

tatctggcgg gcgggatgtt tttacaatgg ctgctggggc cgctgtcgga tcgtattggt 1320

cgccgtccgg tgatgctggc gggagtggtg tggtttatcg tcacctgtct ggcaatattg 1380

ctggcgcaaa acattgaaca attcaccctg ttgcgcttct tgcagggcat aagcctctgt 1440

ttcattggcg ctgtgggata cgccgcaatt caggaatcct tcgaagaggc ggtttgtatc 1500

aagatcaccg cgctgatggc gaacgtggcg ctgattgctc cgctacttgg tccgctggtg 1560

ggcgcggcgt ggatccatgt gctgccctgg gaggggatgt ttgttttgtt tgccgcattg 1620

gcagcgatct cctttttcgg tctgcaacga gccatgcctg aaaccgccac gcgtataggc 1680

gagaaactgt cactgaaaga actcggtcgt gactataagc tggtgctgaa gaacggccgc 1740

tttgtggcgg gggcgctggc gctgggattc gttagtctgc cgttgctggc gtggatcgcc 1800

cagtcgccga ttatcatcat taccggcgag cagttgagca gctatgaata tggcttgctg 1860

caagtgccta ttttcggggc gttaattgcg ggtaacttgc tgttagcgcg tctgacctcg 1920

cgccgcaccg tacgttcgct gattattatg ggcggctggc cgattatgat tggtctattg 1980

gtcgctgctg cggcaacggt tatctcatcg cacgcgtatt tatggatgac tgccgggtta 2040

agtatttatg ctttcggtat tggtctggcg aatgcgggac tggtgcgatt aaccctgttt 2100

gccagcgata tgagtaaagg tacggtttct gccgcgatgg gaatgctgca aatgctgatc 2160

tttaccgttg gtattgaaat cagcaaacat gcctggctga acgggggcaa cggactgttt 2220

aatctcttca accttgtcaa cggaattttg tggctgtcgc tgatggttat ctttttaaaa 2280

gataaacaga tgggaaattc tcacgaaggg taaaaaaatg cctgactgct ttgtgcgatc 2340

aggcattctc gaattaatgg tgatggtcgt caatctgtcg cgattacacc aacgccccgt 2400

aaatcgtcag tagtgcaatc accaccacga ccacgaacga ggttttcttc gccattgaaa 2460

ccgctgcctt cggcgtctcc accttatcga catgcggttc acgcgccaga gagaactgcg 2520

ccagacgcgt taacacctga tactgcgaag tatggaaatc acccagcgaa gcaaaccagg 2580

ccggtaacgc tttctcacca tgaccgatca aggcatatac cacacccgca agacgaaccg 2640

gcacccaatc cagtacatga agcacggcat caatgccgga ctgtaaacga tgatgcggcg 2700

tctgatatcg cgccagccag tattgccatg cacgcaaaaa cgcatacccc atcagcgtaa 2760

cgggtcccca ggttcccccc acaatcagcc agaacagcgg tgcaagataa aaacgaaagt 2820

taatccacag caatgcattt tgcagctcac gcaaatactc acgttcgtcg cagcctgccg 2880

ggacgccgtg aatcatggtg agttcgccag ccatcgtggc acgggcatgg ctatcattac 2940

gtgaagcagc tgtcagataa gcatgataat gaagacgaac tttacctgcg ccaatacaca 3000

gcaaaccaat cagcagccac accagtagcg tgggaacgtt gaacaatact ccctgcaatg 3060

cgcgtaacag tctagagtcg accggtggcg aatgggacgc gccctgtagc ggcgcattaa 3120

<210> 21
<211> 3735
<212> DNA
<213> Escherichia coli

<220>
<221> misc_feature
<222> (1)..(3735)
<223> Dmtr::mdfA; construct for integration of mdfA in delta mtr

<400> 21
agggcagggt cgttaaatag ccgcttatgt ctattgctgg tctcggtacc cggggatcgc 60

ggccgccagt gtgatggata tctgcagaat tcgcccttgc tggcagaaca ccacaatatg 120

actggcattg ccagtgctgc cacgtcggta ttgatcggct atctggcggc gaataccacc 180

acgctgcatc tggggtctgg cggcgtgatg ttgcctaacc actcaccgtt ggtcattgca 240

gaacagttcg gcacgcttaa tacactctat ccggggcgaa tcgatttggg gctgggtcgt 300

gctccgggta gtgaccaacg gacaatgatg gcgctacgtc gtcatatgag cggcgatatt 360

gataatttcc cccgcgatgt ggcggagctg gtggactggt ttgacgcccg cgatcccaat 420

ccgcatgtgc gcccggtacc aggctatggc gagaaaatcc ccgtgtggtt gttaggctcc 480

agcctttaca gcgcgcaact ggcggcgcag cttggtctgc cgtttgcgtt tgcctcacac 540

ttcgcgccgg atatgctgtt ccaggcgctg catctttatc gcagcaactt caaaccgtca 600

gcacggctgg aaaaaccata cgcgatggtg tgcatcaata ttatcgccgc cgacagcaac 660

cgcgacgctg aatttctgtt tacctcaatg cagcaagcct ttgtgaagct gcgccgtggc 720

gaaaccgggc aactgccgcc gccgattcaa aatatggatc agttctggtc accgtctgag 780

cagtatggcg tgcagcaggc gctgagtatg tcgttggtag gtgataaagc gaaagtgcgt 840

catggcttgc agtcgatcct gcgcgaaacc gacgccgatg agattatggt caacgggcag 900

attttcgacc accaggcgcg gctgcattcg tttgagctgg cgatggatgt taaggaagag 960

ttgttgggat agtgtgtctt aacgcgggaa gccttatccg agctggcaac gctgtcctac 1020

atagacctga taagcgaagc gcatcaggca ttgtgtaggc agcagaaatg tcggataagg 1080

caccgctgat tactctagat tcgcgacaga ttgacgacca tcaccattaa ttcgagaatg 1140

cctgatcgca caaagcagtc aggcattttt ttacccttcg tgagaatttc ccatctgttt 1200

atcttttaaa aagataacca tcagcgacag ccacaaaatt ccgttgacaa ggttgaagag 1260

attaaacagt ccgttgcccc cgttcagcca ggcatgtttg ctgatttcaa taccaacggt 1320

aaagatcagc atttgcagca ttcccatcgc ggcagaaacc gtacctttac tcatatcgct 1380

ggcaaacagg gttaatcgca ccagtcccgc attcgccaga ccaataccga aagcataaat 1440

acttaacccg gcagtcatcc ataaatacgc gtgcgatgag ataaccgttg ccgcagcagc 1500

gaccaataga ccaatcataa tcggccagcc gcccataata atcagcgaac gtacggtgcg 1560

gcgcgaggtc agacgcgcta acagcaagtt acccgcaatt aacgccccga aaataggcac 1620

ttgcagcaag ccatattcat agctgctcaa ctgctcgccg gtaatgatga taatcggcga 1680

ctgggcgatc cacgccagca acggcagact aacgaatccc agcgccagcg cccccgccac 1740

aaagcggccg ttcttcagca ccagcttata gtcacgaccg agttctttca gtgacagttt 1800

ctcgcctata cgcgtggcgg tttcaggcat ggctcgttgc agaccgaaaa aggagatcgc 1860

tgccaatgcg gcaaacaaaa caaacatccc ctcccagggc agcacatgga tccacgccgc 1920

gcccaccagc ggaccaagta gcggagcaat cagcgccacg ttcgccatca gcgcggtgat 1980

cttgatacaa accgcctctt cgaaggattc ctgaattgcg gcgtatccca cagcgccaat 2040

gaaacagagg cttatgccct gcaagaagcg caacagggtg aattgttcaa tgttttgcgc 2100

cagcaatatt gccagacagg tgacgataaa ccacaccact cccgccagca tcaccggacg 2160

gcgaccaata cgatccgaca gcggccccag cagccattgt aaaaacatcc cgcccgccag 2220

atacgcggtc atcgaagtag gaacccaatc aatgcccgcc tgatattgtt ccaccacggc 2280

caacataccg ggttgaatca tatcgttgcc gatataggtt gaaaattcgt aaagcaccag 2340

acagagaggg aaaagtaacg cctgacgtcc aagcctggca ccggaagcta atttattttg 2400

catgcaattt cttcgccaat aataatcgcg cagagtttaa taaaagcgca gctaacgaga 2460

aagcgaattt tgtagctgaa accacggtta agcacattct tacattatta cgagtatagc 2520

tacgctttct ttaagtttta tttaacctat gcccgttaca atcacccacc gtaaacaggc 2580

cgcttgaggg aaataagacg atgccgcttt acccagttta acctgcactt tattctcaac 2640

gacttgcctg tatcgcgagg gcttctctcc agtgaaaaat agtgcgactg cgttgttatg 2700

cattgcactg taccagtaca cgagtacaaa agacagaaaa aaagccccga tggtaaaaat 2760

cggggctgta tatattattt tacagattgt gttcgctgtt cagcgatgat tacgcatcac 2820

caccgaaacg acgacgaccg gtagaatcat cacgacgcgg agcgcggcct tcacgacgtt 2880

cgccgctaaa acgacgacca tcaccacggc caccttcacg gcgttcaccg ctgaagttac 2940

gaccgccttc acgacgttcg ccaccgaaac cacgaccacc gccacgacgc tcaccgccag 3000

tatgcggctg tgcatcgccc agtaactgca tgttcatcgg cttgttgaga atgcgagtgc 3060

gcgtaaagtg ttgcagcact tcacccggca tacctttcgg cagttcgatg gtggagtgag 3120

aagcaaacag cttgatgtta ccaatgtaac ggctgctgat gtcgccttcg ttagcaatcg 3180

caccaacgat atgacgaact tcaacaccat catcgcggcc cacttcaatg cggtacagct 3240

gcatatcgcc aacatcacga cgttcacgac gcggacgatc ttcacggtca ccacgcgggc 3300

cacggtcgtt acgatcgcgc ggaccacggt catcacggtc acggaattca cgtttcggac 3360

gcatcggcgc atctggcggt acgatcagag tacgttcacc ctgtgccatt ttcagcagtg 3420

ccgcagccag agtttcgaga tccagctctt caccttcagc agtcggctga attttgctca 3480

gcagtgcgcg gtattgatcc agatcgctgc tttccagctg ctgctgtact ttagcggcga 3540

atttttccag acggcgtttg cctagcagtt ctgcgttcgg cagttctact tccggaatag 3600

tcagcttcat agtacgttca atgttgcgca gcagacgacg ctcgcggttc tcaaaagggc 3660

gaattccagc acactggcgg ccgttactag agtcgaccgg tggcgaatgg gacgcgccct 3720

gtagcggcgc attaa 3735

<210> 22
<211> 1436
<212> DNA
<213> Escherichia coli

<220>
<221> misc_feature
<222> (1)..(1436)
<223> delta aroP on chromosome

<400> 22
cctggcgacg aagcaacaag cccttcgctt cgagacgttg aatcgcctca cgcaaggagg 60

gacgggagac gtcaaactgt tttgccagtt cgcgttccgg tgggagtttt tcgcccgggc 120

ggagagtgcc ttcgaggatc aaaaactcca gttgctgctc aatcacatcg gagagttttg 180

gttggcggat tttgctgtag gccatgagtt cctgtcttaa gccacttgcc gaagtcaatt 240

ggtcttacca atttcatgtc tgtgacgcta aagtaacaaa gtattcacct tatgtccata 300

caggttttga ttgaaatcat gaaactgtgc acattttaac aacttgacat atataacgtt 360

tcaaagttgt aactatgcac aaatgtagac tttacgtagg aaaggagttt tttaacgatt 420

attaaactat aaaaatccga attgaaccga ttcacttacc aattttgtga tttttaattc 480

aattaaaacg aatttaaatt cattctacat attgagaggg gttgaggctg agctttacaa 540

acggtttctt tttaagcaac tcatcttcaa ccatgcataa agcgggtgca ttcgctgccg 600

cataccatta ttcttgatct gacggaagtc tttttgtaac aattcaaact tctttgatgt 660

aaacaaatta atacaacaaa cggaattgca aacttacaca cgcatcactg cgtagatcaa 720

aaaaacaacc accgcacgag gttcgcgatt acaccaacgc cccgtaaatc gtcagtagtg 780

caatcaccac cacgaccacg aacgaggttt tcttcgccat tgaaaccgct gccttcggcg 840

tctccacctt atcgacatgc ggttcacgcg ccagagagaa ctgcgccaga cgcgttaaca 900

cctgatactg cgaagtatgg aaatcaccca gcgaagcaaa ccaggccggt aacgctttct 960

caccatgacc gatcaaggca tataccacac ccgcaagacg aaccggcacc caatccagta 1020

catgaagcac ggcatcaatg ccggactgta aacgatgatg cggcgtctga tatcgcgcca 1080

gccagtattg ccatgcacgc aaaaacgcat accccatcag cgtaacgggt ccccaggttc 1140

cccccacaat cagccagaac agcggtgcaa gataaaaacg aaagttaatc cacagcaatg 1200

cattttgcag ctcacgcaaa tactcacgtt cgtcgcagcc tgccgggacg ccgtgaatca 1260

tggtgagttc gccagccatc gtggcacggg catggctatc attacgtgaa gcagctgtca 1320

gataagcatg ataatgaaga cgaactttac ctgcgccaat acacagcaaa ccaatcagca 1380

gccacaccag tagcgtggga acgttgaaca atactccctg caatgcgcgt aacagt 1436

<210> 23
<211> 2988
<212> DNA
<213> Escherichia coli

<220>
<221> misc_feature
<222> (1)..(2988)
<223> mdfA in delta aroP on chromosome

<400> 23
cctggcgacg aagcaacaag cccttcgctt cgagacgttg aatcgcctca cgcaaggagg 60

gacgggagac gtcaaactgt tttgccagtt cgcgttccgg tgggagtttt tcgcccgggc 120

ggagagtgcc ttcgaggatc aaaaactcca gttgctgctc aatcacatcg gagagttttg 180

gttggcggat tttgctgtag gccatgagtt cctgtcttaa gccacttgcc gaagtcaatt 240

ggtcttacca atttcatgtc tgtgacgcta aagtaacaaa gtattcacct tatgtccata 300

caggttttga ttgaaatcat gaaactgtgc acattttaac aacttgacat atataacgtt 360

tcaaagttgt aactatgcac aaatgtagac tttacgtagg aaaggagttt tttaacgatt 420

attaaactat aaaaatccga attgaaccga ttcacttacc aattttgtga tttttaattc 480

aattaaaacg aatttaaatt cattctacat attgagaggg gttgaggctg agctttacaa 540

acggtttctt tttaagcaac tcatcttcaa ccatgcataa agcgggtgca ttcgctgccg 600

cataccatta ttcttgatct gacggaagtc tttttgtaac aattcaaact tctttgatgt 660

aaacaaatta atacaacaaa cggaattgca aacttacaca cgcatcactg cgtagatcaa 720

aaaaacaacc accgcacgag gttcgcgata caggcaagtc gttgagaata aagtgcaggt 780

taaactgggt aaagcggcat cgtcttattt ccctcaagcg gcctgtttac ggtgggtgat 840

tgtaacgggc ataggttaaa taaaacttaa agaaagcgta gctatactcg taataatgta 900

agaatgtgct taaccgtggt ttcagctaca aaattcgctt tctcgttagc tgcgctttta 960

ttaaactctg cgcgattatt attggcgaag aaattgcatg caaaataaat tagcttccgg 1020

tgccaggctt ggacgtcagg cgttactttt ccctctctgt ctggtgcttt acgaattttc 1080

aacctatatc ggcaacgata tgattcaacc cggtatgttg gccgtggtgg aacaatatca 1140

ggcgggcatt gattgggttc ctacttcgat gaccgcgtat ctggcgggcg ggatgttttt 1200

acaatggctg ctggggccgc tgtcggatcg tattggtcgc cgtccggtga tgctggcggg 1260

agtggtgtgg tttatcgtca cctgtctggc aatattgctg gcgcaaaaca ttgaacaatt 1320

caccctgttg cgcttcttgc agggcataag cctctgtttc attggcgctg tgggatacgc 1380

cgcaattcag gaatccttcg aagaggcggt ttgtatcaag atcaccgcgc tgatggcgaa 1440

cgtggcgctg attgctccgc tacttggtcc gctggtgggc gcggcgtgga tccatgtgct 1500

gccctgggag gggatgtttg ttttgtttgc cgcattggca gcgatctcct ttttcggtct 1560

gcaacgagcc atgcctgaaa ccgccacgcg tataggcgag aaactgtcac tgaaagaact 1620

cggtcgtgac tataagctgg tgctgaagaa cggccgcttt gtggcggggg cgctggcgct 1680

gggattcgtt agtctgccgt tgctggcgtg gatcgcccag tcgccgatta tcatcattac 1740

cggcgagcag ttgagcagct atgaatatgg cttgctgcaa gtgcctattt tcggggcgtt 1800

aattgcgggt aacttgctgt tagcgcgtct gacctcgcgc cgcaccgtac gttcgctgat 1860

tattatgggc ggctggccga ttatgattgg tctattggtc gctgctgcgg caacggttat 1920

ctcatcgcac gcgtatttat ggatgactgc cgggttaagt atttatgctt tcggtattgg 1980

tctggcgaat gcgggactgg tgcgattaac cctgtttgcc agcgatatga gtaaaggtac 2040

ggtttctgcc gcgatgggaa tgctgcaaat gctgatcttt accgttggta ttgaaatcag 2100

caaacatgcc tggctgaacg ggggcaacgg actgtttaat ctcttcaacc ttgtcaacgg 2160

aattttgtgg ctgtcgctga tggttatctt tttaaaagat aaacagatgg gaaattctca 2220

cgaagggtaa aaaaatgcct gactgctttg tgcgatcagg cattctcgaa ttaatggtga 2280

tggtcgtcaa tctgtcgcga ttacaccaac gccccgtaaa tcgtcagtag tgcaatcacc 2340

accacgacca cgaacgaggt tttcttcgcc attgaaaccg ctgccttcgg cgtctccacc 2400

ttatcgacat gcggttcacg cgccagagag aactgcgcca gacgcgttaa cacctgatac 2460

tgcgaagtat ggaaatcacc cagcgaagca aaccaggccg gtaacgcttt ctcaccatga 2520

ccgatcaagg catataccac acccgcaaga cgaaccggca cccaatccag tacatgaagc 2580

acggcatcaa tgccggactg taaacgatga tgcggcgtct gatatcgcgc cagccagtat 2640

tgccatgcac gcaaaaacgc ataccccatc agcgtaacgg gtccccaggt tccccccaca 2700

atcagccaga acagcggtgc aagataaaaa cgaaagttaa tccacagcaa tgcattttgc 2760

agctcacgca aatactcacg ttcgtcgcag cctgccggga cgccgtgaat catggtgagt 2820

tcgccagcca tcgtggcacg ggcatggcta tcattacgtg aagcagctgt cagataagca 2880

tgataatgaa gacgaacttt acctgcgcca atacacagca aaccaatcag cagccacacc 2940

agtagcgtgg gaacgttgaa caatactccc tgcaatgcgc gtaacagt 2988

<210> 24
<211> 2004
<212> DNA
<213> Escherichia coli


<220>
<221> misc_feature
<222> (1)..(2004)
<223> delta mtr on chromosome

<400> 24
gctggcagaa caccacaata tgactggcat tgccagtgct gccacgtcgg tattgatcgg 60

ctatctggcg gcgaatacca ccacgctgca tctggggtct ggcggcgtga tgttgcctaa 120

ccactcaccg ttggtcattg cagaacagtt cggcacgctt aatacactct atccggggcg 180

aatcgatttg gggctgggtc gtgctccggg tagtgaccaa cggacaatga tggcgctacg 240

tcgtcatatg agcggcgata ttgataattt cccccgcgat gtggcggagc tggtggactg 300

gtttgacgcc cgcgatccca atccgcatgt gcgcccggta ccaggctatg gcgagaaaat 360

ccccgtgtgg ttgttaggct ccagccttta cagcgcgcaa ctggcggcgc agcttggtct 420

gccgtttgcg tttgcctcac acttcgcgcc ggatatgctg ttccaggcgc tgcatcttta 480

tcgcagcaac ttcaaaccgt cagcacggct ggaaaaacca tacgcgatgg tgtgcatcaa 540

tattatcgcc gccgacagca accgcgacgc tgaatttctg tttacctcaa tgcagcaagc 600

ctttgtgaag ctgcgccgtg gcgaaaccgg gcaactgccg ccgccgattc aaaatatgga 660

tcagttctgg tcaccgtctg agcagtatgg cgtgcagcag gcgctgagta tgtcgttggt 720

aggtgataaa gcgaaagtgc gtcatggctt gcagtcgatc ctgcgcgaaa ccgacgccga 780

tgagattatg gtcaacgggc agattttcga ccaccaggcg cggctgcatt cgtttgagct 840

ggcgatggat gttaaggaag agttgttggg atagtgtgtc ttaacgcggg aagccttatc 900

cgagctggca acgctgtcct acatagacct gataagcgaa gcgcatcagg cattgtgtag 960

gcagcagaaa tgtcggataa ggcaccgctg attactctag attcgcgagg gcttctctcc 1020

agtgaaaaat agtgcgactg cgttgttatg cattgcactg taccagtaca cgagtacaaa 1080

agacagaaaa aaagccccga tggtaaaaat cggggctgta tatattattt tacagattgt 1140

gttcgctgtt cagcgatgat tacgcatcac caccgaaacg acgacgaccg gtagaatcat 1200

cacgacgcgg agcgcggcct tcacgacgtt cgccgctaaa acgacgacca tcaccacggc 1260

caccttcacg gcgttcaccg ctgaagttac gaccgccttc acgacgttcg ccaccgaaac 1320

cacgaccacc gccacgacgc tcaccgccag tatgcggctg tgcatcgccc agtaactgca 1380

tgttcatcgg cttgttgaga atgcgagtgc gcgtaaagtg ttgcagcact tcacccggca 1440

tacctttcgg cagttcgatg gtggagtgag aagcaaacag cttgatgtta ccaatgtaac 1500

ggctgctgat gtcgccttcg ttagcaatcg caccaacgat atgacgaact tcaacaccat 1560

catcgcggcc cacttcaatg cggtacagct gcatatcgcc aacatcacga cgttcacgac 1620

gcggacgatc ttcacggtca ccacgcgggc cacggtcgtt acgatcgcgc ggaccacggt 1680

catcacggtc acggaattca cgtttcggac gcatcggcgc atctggcggt acgatcagag 1740

tacgttcacc ctgtgccatt ttcagcagtg ccgcagccag agtttcgaga tccagctctt 1800

caccttcagc agtcggctga attttgctca gcagtgcgcg gtattgatcc agatcgctgc 1860

tttccagctg ctgctgtact ttagcggcga atttttccag acggcgtttg cctagcagtt 1920

ctgcgttcgg cagttctact tccggaatag tcagcttcat agtacgttca atgttgcgca 1980

gcagacgacg ctcgcggttc tcaa 2004

<210> 25
<211> 3553
<212> DNA
<213> Escherichia coli

<220>
<221> misc_feature
<222> (1)..(3553)
<223> mdfA in delta mtr on chromosome

<400> 25
ctggcagaac accacaatat gactggcatt gccagtgctg ccacgtcggt attgatcggc 60

tatctggcgg cgaataccac cacgctgcat ctggggtctg gcggcgtgat gttgcctaac 120

cactcaccgt tggtcattgc agaacagttc ggcacgctta atacactcta tccggggcga 180

atcgatttgg ggctgggtcg tgctccgggt agtgaccaac ggacaatgat ggcgctacgt 240

cgtcatatga gcggcgatat tgataatttc ccccgcgatg tggcggagct ggtggactgg 300

tttgacgccc gcgatcccaa tccgcatgtg cgcccggtac caggctatgg cgagaaaatc 360

cccgtgtggt tgttaggctc cagcctttac agcgcgcaac tggcggcgca gcttggtctg 420

ccgtttgcgt ttgcctcaca cttcgcgccg gatatgctgt tccaggcgct gcatctttat 480

cgcagcaact tcaaaccgtc agcacggctg gaaaaaccat acgcgatggt gtgcatcaat 540

attatcgccg ccgacagcaa ccgcgacgct gaatttctgt ttacctcaat gcagcaagcc 600

tttgtgaagc tgcgccgtgg cgaaaccggg caactgccgc cgccgattca aaatatggat 660

cagttctggt caccgtctga gcagtatggc gtgcagcagg cgctgagtat gtcgttggta 720

ggtgataaag cgaaagtgcg tcatggcttg cagtcgatcc tgcgcgaaac cgacgccgat 780

gagattatgg tcaacggcca gattttcgac caccaggcgc ggctgcattc gtttgagctg 840

gcgatggatg ttaaggaaga gttgttggga tagtgtgtct taacgcggga agccttatcc 900

gagctggcaa cgctgtccta catagacctg ataagcgaag cgcatcaggc attgtgtagg 960

cagcagaaat gtcggataag gcaccgctga ttactctaga ttcgcgacag attgacgacc 1020

atcaccatta attcgagaat gcctgatcgc acaaagcagt caggcatttt tttacccttc 1080

gtgagaattt cccatctgtt tatcttttaa aaagataacc atcagcgaca gccacaaaat 1140

tccgttgaca aggttgaaga gattaaacag tccgttgccc ccgttcagcc aggcatgttt 1200

gctgatttca ataccaacgg taaagatcag catttgcagc attcccatcg cggcagaaac 1260

cgtaccttta ctcatatcgc tggcaaacag ggttaatcgc accagtcccg cattcgccag 1320

accaataccg aaagcataaa tacttaaccc ggcagtcatc cataaatacg cgtgcgatga 1380

gataaccgtt gccgcagcag cgaccaatag accaatcata atcggccagc cgcccataat 1440

aatcagcgaa cgtacggtgc ggcgcgaggt cagacgcgct aacagcaagt tacccgcaat 1500

taacgccccg aaaataggca cttgcagcaa gccatattca tagctgctca actgctcgcc 1560

ggtaatgatg ataatcggcg actgggcgat ccacgccagc aacggcagac taacgaatcc 1620

cagcgccagc gcccccgcca caaagcggcc gttcttcagc accagcttat agtcacgacc 1680

gagttctttc agtgacagtt tctcgcctat acgcgtggcg gtttcaggca tggctcgttg 1740

cagaccgaaa aaggagatcg ctgccaatgc ggcaaacaaa acaaacatcc cctcccaggg 1800

cagcacatgg atccacgccg cgcccaccag cggaccaagt agcggagcaa tcagcgccac 1860

gttcgccatc agcgcggtga tcttgataca aaccgcctct tcgaaggatt cctgaattgc 1920

ggcgtatccc acagcgccaa tgaaacagag gcttatgccc tgcaagaagc gcaacagggt 1980

gaattgttca atgttttgcg ccagcaatat tgccagacag gtgacgataa accacaccac 2040

tcccgccagc atcaccggac ggcgaccaat acgatccgac agcggcccca gcagccattg 2100

taaaaacatc ccgcccgcca gatacgcggt catcgaagta ggaacccaat caatgcccgc 2160

ctgatattgt tccaccacgg ccaacatacc gggttgaatc atatcgttgc cgatataggt 2220

tgaaaattcg taaagcacca gacagagagg gaaaagtaac gcctgacgtc caagcctggc 2280

accggaagct aatttatttt gcatgcaatt tcttcgccaa taataatcgc gcagagttta 2340

ataaaagcgc agctaacgag aaagcgaatt ttgtagctga aaccacggtt aagcacattc 2400

ttacattatt acgagtatag ctacgctttc tttaagtttt atttaaccta tgcccgttac 2460

aatcacccac cgtaaacagg ccgcttgagg gaaataagac gatgccgctt tacccagttt 2520

aacctgcact ttattctcaa cgacttgcct gtatcgcgag ggcttctctc cagtgaaaaa 2580

tagtgcgact gcgttgttat gcattgcact gtaccagtac acgagtacaa aagacagaaa 2640

aaaagccccg atggtaaaaa tcggggctgt atatattatt ttacagattg tgttcgctgt 2700

tcagcgatga ttacgcatca ccaccgaaac gacgacgacc ggtagaatca tcacgacgcg 2760

gagcgcggcc ttcacgacgt tcgccgctaa aacgacgacc atcaccacgg ccaccttcac 2820

ggcgttcacc gctgaagtta cgaccgcctt cacgacgttc gccaccgaaa ccacgaccac 2880

cgccacgacg ctcaccgcca gtatgcggct gtgcatcgcc cagtaactgc atgttcatcg 2940

gcttgttgag aatgcgagtg cgcgtaaagt gttgcagcac ttcacccggc atacctttcg 3000

gcagttcgat ggtggagtga gaagcaaaca gcttgatgtt accaatgtaa cggctgctga 3060

tgtcgccttc gttagcaatc gcaccaacga tatgacgaac ttcaacacca tcatcgcggc 3120

ccacttcaat gcggtacagc tgcatatcgc caacatcacg acgttcacga cgcggacgat 3180

cttcacggtc accacgcggg ccacggtcgt tacgatcgcg cggaccacgg tcatcacggt 3240

cacggaattc acgtttcgga cgcatcggcg catctggcgg tacgatcaga gtacgttcac 3300

cctgtgccat tttcagcagt gccgcagcca gagtttcgag atccagctct tcaccttcag 3360

cagtcggctg aattttgctc agcagtgcgc ggtattgatc cagatcgctg ctttccagct 3420

gctgctgtac tttagcggcg aatttttcca gacggcgttt gcctagcagt tctgcgttcg 3480

gcagttctac ttccggaata gtcagcttca tagtacgttc aatgttgcgc agcagacgac 3540

gctcgcggtt ctc 3553

201800223 4

## Description

### Field of the invention

The present invention pertains to a new method for the fermentative production of L-tryptophan using a modified microorganism of the *Enterobacteriaceae* family, in which the expression level of the mdfA gene is enhanced.

### Background of the invention

L-tryptophan is used in human medicine and in the pharmaceutical industry, in food industry and in animal nutrition.

L-tryptophan can be produced by fermentation of *Enterobacteriaceae* strains, especially *Escherichia coli* (*E. coli*) and *Serratia marcescens.* Because of the great significance, work is constantly being done on improving the methods of production.

Methodological improvements may concern measures relating to fermentation technology, such as, for example, stirring and oxygen supply, or the composition of the nutrient media, such as, for example, selection of the sugar used or the sugar concentration during the fermentation, or the working-up to the product form, for example by means of ion exchange chromatography, or the intrinsic performance properties of the microorganism itself.

In wild-type strains, strict regulatory mechanisms prevent metabolic products such as amino acids from being produced in excess of what is needed by said strains and from being released into the medium. The construction of amino acid-overproducing strains therefore requires, from a manufacturer's point of view, these metabolic regulations to be overcome.

Methods of mutagenesis, selection and mutant choice are used for removing said control mechanisms and improving the performance properties of these microorganisms.

Owing to the complexity of the biosynthesis pathway of aromatic L-amino acids like L-tryptophan and owing to the interconnection thereof with many other metabolic pathways in the cell, it is not always possible to predict which genetic variations or modifications of a strain can achieve an improved production of L-tryptophan.

The multidrug transporter MdfA is known as an efflux pump driven by the proton motive force (Lewinson et al., Proc. Natl. Acad. Sci. U.S.A. 100 (4) 1667-1672). Its additional function as a Na⁺/H⁺ antiporter and its involvement in the production and accumulation of amino acids, such as L-lysine, L-arginine, L-threonine or L-histidine, has been reported in WO 2007/069782 A1. Therein, a bacterium of the *Enterobacteriaceae* family with increased expression level of a gene selected from the group consisting of nhaA, nhaB, nhaR, chaA, mdfA and combinations thereof is disclosed. However, WO 2007/069782 A1 is silent on the influence of an enhanced mdfA expression level on L-tryptophan productivity.

In view of the above, it was a remaining need to further optimize especially those strains of the *Enterobacteriaceae* family that are suitable for producing L-tryptophan, and to provide improved methods for the fermentative production of L-tryptophan, using these microorganisms of the *Enterobacteriaceae* family. Further, it was particularly desirable to establish new techniques enabling the modulation of the expression level of genes involved in transport processes of aromatic compounds as toxicity caused by (even low amounts of) aromatic amino acid derivates and intermediates requires effective transport systems. However, the gene expression levels should be adapted to the specific needs of the abundance of transporter proteins in the cell membrane without affecting cellular fitness and general production capacities.

### Summary of the invention

The present invention provides a method of producing L-tryptophan, the method comprising culturing a L-tryptophan producing microorganism belonging to the *Enterobacteriaceae* family in a fermentation medium; wherein the L-tryptophan producing microorganism has been modified by enhancing the expression level of the mdfA gene or by enhancing the expression level of an mdfA allele.

Further, the present invention pertains to a method for producing an L-tryptophan producing microorganism by transformation, transduction or conjugation, wherein the transformation, transduction or conjugation is done using a vector comprising
a) a polynucleotide sequence comprising the nucleotide sequence of SEQ ID NO.: 1;
b) a polynucleotide that hybridizes with a complementary strand of SEQ ID NO.: 1 under stringent conditions;
c) a naturally occurring mutant or polymorphic form of a polynucleotide according to a) or b);
d) a polynucleotide having a sequence identity of at least 80% of the nucleotide sequence of SEQ ID NO.: 1;
e) a polynucleotide including substitutions, deletions, insertions or additions of 1 to 60 nucleotides in relation to the nucleotide sequence of SEQ ID NO.:1;
f) a polynucleotide encoding a protein comprising the amino acid sequence of SEQ ID NO.: 2;
g) a polynucleotide encoding a protein comprising an amino acid sequence which includes substitution, deletion, insertion or addition of 1 to 20 amino acids in SEQ ID NO.: 2;
and comprising a promoter regulating the expression of polynucleotides a) to g).

In addition, the invention provides a L-tryptophan producing microorganism of the *Enterobacteriaceae* family, wherein the microorganism has been modified by enhancing the expression level of the mdfA gene or by enhancing the expression level of an mdfA allele.

Finally, the invention is directed to the use of the aforementioned microorganism for producing L-tryptophan or, alternatively, for producing a feedstuff additive containing L-tryptophan.

### Detailed description of the invention

In the work forming the basis of the present invention, it was surprisingly found that the L-tryptophan productivity in a L-tryptophan producing strain of the *Enterobacteriaceae* family may be significantly increased by modulating the expression level of the mdfA gene or by modulating the expression level of a mdfA allele, respectively in an appropriate way.

More specifically, the present invention provides a method of producing L-tryptophan, the method comprising culturing a L-tryptophan producing microorganism belonging to the *Enterobacteriaceae* family in a fermentation medium; wherein the L-tryptophan producing microorganism has been modified by enhancing the expression level of the mdfA gene or by enhancing the expression level of an mdfA allele.

For the sake of clarity and completeness, the coding region of the mdfA gene is depicted in SEQ ID NO.: 1. The amino acid sequence of MdfA is depicted in SEQ ID NO.: 2.

An allele is a variant of a given gene.

The mdfA allele according to the present invention may be a polynucleotide selected from the group consisting of:
a) a polynucleotide sequence comprising the nucleotide sequence of SEQ ID NO.: 1;
b) a polynucleotide that hybridizes with a complementary strand of SEQ ID NO.: 1 under stringent conditions;
c) a naturally occurring mutant or polymorphic form of a polynucleotide according to a) or b);
d) a polynucleotide having a sequence identity of at least 80%, at least 85%, or at least 90%, preferably at least 95% of the nucleotide sequence of SEQ ID NO.: 1;
e) a polynucleotide including substitutions, deletions, insertions or additions of 1 to 60 nucleotides in relation to the nucleotide sequence of SEQ ID NO.: 1;
f) a polynucleotide encoding a protein comprising the amino acid sequence of SEQ ID NO.: 2; g) a polynucleotide encoding a protein comprising an amino acid sequence which includes substitution, deletion, insertion or addition of 1 to 20 amino acids in SEQ ID NO.: 2.

The terms "enhancing' or 'enhanced' or 'overexpressed' or 'increased expression level' 'enhanced expression level' or 'overexpression" are used interchangeably in this text and have similar meaning. These terms, in this context, describe the increase in the intracellular activity of an enzymatic activity which is encoded by the corresponding DNA, for example by increasing the number of copies of the gene, using a stronger promoter or using an allele with increased activity and possibly combining these measures.

The expression level of the mdfA gene or of the mdfA allele may be enhanced by increasing the copy number of the gene or allele by at least 1.

To increase the expression level of a gene/allele, copies of genes/alleles are preferably added chromosomally. Chromosomally there may be one or several extra copies on the genome that may be introduced by methods of recombination known to the man skilled in the art. Extra-chromosomal genes may be carried by different types of plasmids or bacterial artificial chromosomes that differ with respect to their origin of replication and thus their copy number in the cell. They may be present as 1-5 copies, about 20 or up to 500 copies, corresponding to low copy number plasmids with tight replication, medium copy number plasmids, or high copy number plasmids.

The copy number of the mdfA gene or of the mdfA allele may be increased by at least 1 by integrating the gene into the chromosome of the microorganism.

The expression level of the mdfA gene or of the mdfA allele may also be enhanced by modifying the expression regulatory sequence of the gene. As indicated before, the mdfA gene or mdfA allele may be combined with a stronger promoter. These promoters may be inducible; they may be homologous or heterologous. The man skilled in the art knows which promoters are the most convenient.

When genes are organized in an operon, it is possible to enhance their expression level by adding one supplementary copy of these genes under control of a single promoter. Expression may also be enhanced by replacing the chromosomal wild-type promoter with an artificial promoter stronger than the wild-type promoter. The expert in the field knows how to determine promoter strength.

The microorganisms according to the present invention and used in the method according to the present invention, respectively, are representatives of the *Enterobacteriaceae* family, selected from the genera *Escherichia, Erwinia* and *Providencia.* The genus *Escherichia* is preferred. The species *Escherichia coli* must be mentioned in particular. Preferably, a microorganism having an increased expression of the Trp operon is used.

The inventors have surprisingly found that a particularly strong L-tryptophan productivity may be achieved in a L-tryptophan producing strain of the *Enterobacteriaceae,* in particular of *Escherichia coli,* in case the mdfA gene or the mdfA allele is integrated into specific chromosomal or extra-chromosomal loci. More specifically, the inventors have found that using the specific chromosomal or extra-chromosomal loci for mdfA integration enables the modulation of the expression level of mdfA in a way that improves the capacity of fermentative production of L-tryptophan when commercially relevant concentrations accumulate in the fermentation broth.

As used in the context of the present invention, the term "modulation of expression level" refers to setting a balanced expression level leading to an optimized yield of the amino acid (L-tryptophan) product.

The expression of a stable integrated gene is strongly influenced by regulatory sequences in the chromosomal environment of the integration site, e.g. combined effects of the start codon and the flanking regions have been described (Stenstrom et al., Gene 273(2):259-65 (2001); Hui et al., EMBO Journal 3(3):623-9 (1984)).

In one embodiment, a copy of the mdfA gene or the mdfA allele with native expression signals is integrated in the chromosomal mtr locus, whereby the mtr gene is deleted simultaneously. Preferably, the mtr promoter remains intact, as indicated in SEQ ID NO.: 25, i.e. the flanking regions probably regulates the expression of the mdfA gene or the mdfA allele.

In addition or as an alternative to the above, a copy of the mdfA gene or of the mdfA allele may be integrated in the chromosomal aroP locus, whereby the aroP gene is deleted simultaneously. Preferably, the aroP promoter remains intact, i.e. the flanking regions probably regulates the expression of the mdfA gene or the mdfA allele.

Accordingly, the mdfA expression level might be adapted to the specific needs of the abundance of MdfA proteins in the cell membrane by using different integration loci, because regulatory sequences of the flanking regions might have impact.

The microorganisms according to the invention and used in the method of the present invention, respectively, can be produced using the above-described methods for transformation, transduction or conjugation. Preferably, the transformation, transduction or conjugation is done using a vector comprising
a) a polynucleotide sequence comprising the nucleotide sequence of SEQ ID NO.: 1;
b) a polynucleotide that hybridizes with a complementary strand of SEQ ID NO.: 1 under stringent conditions;
c) a naturally occurring mutant or polymorphic form of a polynucleotide according to a) or b);
d) a polynucleotide having a sequence identity of at least 80%, at least 85%, or at least 90%, preferably at least 95% of the nucleotide sequence of SEQ ID NO.: 1;
e) a polynucleotide including substitutions, deletions, insertions or additions of 1 to 60 nucleotides in relation to the nucleotide sequence of SEQ ID NO.: 1;
f) a polynucleotide encoding a protein comprising the amino acid sequence of SEQ ID NO.: 2;
g) a polynucleotide encoding a protein comprising an amino acid sequence which includes substitution, deletion, insertion or addition of 1 to 20 amino acids in SEQ ID NO.: 2;
and comprising a promoter regulating the expression of polynucleotides a) to g).

To produce the inventive strains of the *Enterobacteriaceae* family, preference is given to using strains (starting strains or parent strains) already having the capability of enriching L-tryptophan in the cell and/or of secreting it into the nutrient medium surrounding the cell or accumulating it in the fermentation broth. The expression "produce" can also be used for this.

More particularly, the strains used for the measures of the invention have the capability of enriching or of accumulating in the cell and/or in the nutrient medium or the fermentation broth ≥ (at least) 0.25 g/l, ≥ 0.5 g/l, ≥ 1.0 g/l, ≥ 1.5 g/l, ≥ 2.0 g/l, ≥ 4 g/l, ≥ 10 g/l, ≥ 20 g/l, ≥ 30 g/l or ≥ 50 g/l of L-tryptophan, in ≤ (at most) 120 hours, ≤ 96 hours, ≤ 48 hours, ≤ 36 hours, ≤ 24 hours or ≤ 12 hours. Here, the strains can be strains which have been produced by mutagenesis and selection, by recombinant DNA techniques or by a combination of both methods.

The preferably recombinant microorganisms containing the nucleotide sequence according to the present invention can produce L-tryptophan, from glucose, sucrose, lactose, fructose, maltose, molasses, possibly starch, possibly cellulose or from glycerol and ethanol, possibly also from mixtures.

Examples of strains of the genus *Escherichia,* in particular of the species *Escherichia coli,* that are suitable for the parent strain and produce or secrete L-tryptophan are:

| | |
|---|---|
| - Escherichia coli SV164(pGH5) | (WO 94/08031) |
| - E. coli AGX17(pGX44) (NRRL B-12263) | (US 4,371,614) |
| - E. coli AGX6(pGX50)aroP (NRRL B-12264) | (US 4,371,614) |
| - Escherichia coli AGX17/pGX50,pACKG4-pps | (WO 97/08333) |
| - Escherichia coli ATCC 31743 | (CA 1182409) |
| - E. coli C534/pD2310,pDM136 (ATCC 39795) | (WO8 7/01130) |
| - Escherichia coli JB102/p5LRPS2 | (US 5,939,295). |

L-Tryptophan-producing or L-tryptophan-secreting strains from the *Enterobacteriaceae* family preferably have, inter alia, one or more of the genetic or phenotypic features selected from the group consisting of: resistance to 5-methyl-DL-tryptophan, resistance to 5-fluoro-tryptophan, resistance to 4-methyl-DL-tryptophan, resistance to 6-methyl-DL-tryptophan, resistance to 4-fluoro-tryptophan, resistance to 6-fluoro-tryptophan, resistance to anthranilate, resistance to tryptazan, resistance to indole, resistance to indoleacrylic acid, need for phenylalanine, need for tyrosine, possibly capacity for sucrose utilization, enhancement of the tryptophan operon, preferably anthranilate synthase, preferably the feedback-resistant form, a partially defective tryptophanyl-tRNA synthase, an attenuated tryptophan uptake, a defective tryptophanase, inactivated repressor proteins, enhancement of serine biosynthesis, enhancement of phophoenolpyruvate synthesis, enhancement of D-erythrose 4-phosphate synthesis, enhancement of 3-deoxy-D-arabino-heptulosonate 7-phosphate (DHAP) synthesis, enhancement of chorismate biosynthesis.

Furthermore, for the production of L-tryptophan with strains of the *Enterobacteriaceae* family it may be advantageous to additionally enhance one or more enzymes of the known biosynthesis pathways or enzymes of anaplerotic metabolism or enzymes for the production of reduced nicotinamide adenine dinucleotide phosphate or enzymes of glycolysis or PTS enzymes or enzymes of sulphur metabolism. The use of endogenous genes is generally preferred.

Furthermore, for the production of L-tryptophan, it may be advantageous to additionally switch off undesired secondary reactions (Nakayama: "Breeding of Amino Acid Producing Microorganisms", in: Overproduction of Microbial Products, Krumphanzl, Sikyta, Vanek (eds.), Academic Press, London, UK, 1982).

In accordance with the above, the present invention further provides an L-tryptophan producing microorganism of the *Enterobacteriaceae* family, whereby the microorganism has been modified by enhancing the expression level of the mdfA gene or by enhancing the expression level of an mdfA allele.

In one embodiment, the mdfA allele is a polynucleotide selected from the group consisting of:
a) a polynucleotide sequence comprising the nucleotide sequence of SEQ ID NO.: 1;
b) a polynucleotide that hybridizes with a complementary strand of SEQ ID NO.: 1 under stringent conditions;
c) a naturally occurring mutant or polymorphic form of a polynucleotide according to a) or b);
d) a polynucleotide having a sequence identity of at least 80%, at least 85%, or at least 90%, preferably at least 95% of the nucleotide sequence of SEQ ID NO.: 1;
e) a polynucleotide including substitutions, deletions, insertions or additions of 1 to 60 nucleotides in relation to the nucleotide sequence of SEQ ID NO.: 1;
f) a polynucleotide encoding a protein comprising the amino acid sequence of SEQ ID NO.: 2;
g) a polynucleotide encoding a protein comprising an amino acid sequence which includes substitution, deletion, insertion or addition of 1 to 20 amino acids in SEQ ID NO.: 2.

For enhancing the mdfA expression, the mdfA gene/allele is preferably integrated in the chromosomal mtr locus. In one specific embodiment, a copy of the mdfA gene or of the mdfA allele is integrated in the mtr locus with simultaneous deletion of the mtr gene. Optionally, the chromosomal environment of the mtr locus remains intact and regulates the expression of the mdfA gene or the mdfA allele integrated in the mtr locus.

The aforementioned L-tryptophan producing microorganism may be used for producing L- tryptophan or, alternatively, for producing a feedstuff additive containing L-tryptophan.

The microorganisms according to the invention and used in the method according to the present invention, respectively are cultivated in the batch process, in the fed-batch process, in the repeated fed-batch process or in a continuous process (DE102004028859.3 or US 5,763,230). Overviews of such processes are available in the textbook by Chmiel (Bioprozesstechnik 1. Einführung in die Bioverfahrenstechnik [Bioprocess technology 1. Introduction to bioprocess technology] (Gustav Fischer Verlag, Stuttgart, 1991)) or in the textbook by Storhas (Bioreaktoren und periphere Einrichtungen [Bioreactors and peripheral devices] (Vieweg Verlag, Braunschweig/Wiesbaden, 1994)).

In the case of a batch process, all starting materials, with some exceptions, such as oxygen and pH correcting agent for example, are initially charged in the form of a batch and the microorganism is cultured in the medium obtained.

In the case of a fed-batch process, the microorganism is initially cultured by means of a batch process (batch phase). This is followed by adding to the culture in a continuous or discontinuous manner a starting material essential for the production of the product, also multiple starting materials if necessary (feed phase).

In the case of a repeated fed-batch process, what is involved is a fed-batch process in which, after completion of fermentation, a portion of the fermentation broth obtained serves as inoculum for starting a fresh repeated fed-batch process. This cycle can be repeated multiple times if necessary. Repeated fed-batch processes are, for example, described in WO 02/18543 and WO 05/014843.

In the case of a continuous process, a batch or fed-batch process is followed by continuously adding one or more, possibly all, starting materials to the culture and simultaneously removing fermentation broth. Continuous processes are, for example, described in the patent specifications US 5,763,230, WO 05/014840, WO 05/014841 and WO 05/014842. The culture medium has to satisfy the demands of the particular strains in a suitable manner. Culture media of different microorganisms are described in the handbook "Manual of Methods for General Bacteriology" of the American Society for Bacteriology (Washington D.C., USA, 1981). The terms culture medium, fermentation medium and nutrient medium or medium are interchangeable.

In general, a culture medium contains, inter alia, one or more carbon source(s), nitrogen source(s) and phosphorus source(s).

It is possible to use, as carbon source, sugars and carbohydrates such as, for example, glucose, sucrose, lactose, fructose, maltose, molasses, starch and possibly cellulose, oils and fats such as, for example, soybean oil, sunflower oil, arachis oil and coconut fat, fatty acids such as, for example, palmitic acid, stearic acid and linoleic acid, alcohols such as, for example, glycerol and ethanol and organic acids such as, for example, acetic acid. These substances may be used individually or as a mixture.

It is possible to use, as nitrogen source, organic nitrogen-containing compounds such as peptones, yeast extract, meat extract, malt extract, corn steep liquor, soybean flour and urea, or inorganic compounds such as ammonium sulphate, ammonium chloride, ammonium phosphate, ammonium carbonate and ammonium nitrate. The nitrogen sources may be used individually or as a mixture.

It is possible to use, as phosphorus source, phosphoric acid, potassium dihydrogen phosphate or dipotassium hydrogen phosphate or the corresponding sodium-containing salts.

Furthermore, the culture medium must contain salts of metals, such as magnesium sulphate or iron sulphate for example, that are necessary for growth. Finally, essential growth substances such as amino acids and vitamins may be used in addition to the substances mentioned above. Moreover, suitable precursors may be added to the culture medium. The aforementioned starting materials may be added to the culture in the form of a single batch or be appropriately fed in during cultivation.

The fermentation is generally carried out at a pH of from 5.5 to 9.0, more particularly from 6.0 to 8.0. To control the pH of the culture, appropriate use is made of basic compounds such as sodium hydroxide, potassium hydroxide, ammonia or aqueous ammonia or acidic compounds such as phosphoric acid or sulphuric acid. To control the evolution of foam, it is possible to use antifoams such as, for example, fatty acid polyglycol esters. To maintain the stability of plasmids, it is possible to add to the medium suitable selective substances, for example antibiotics. To maintain aerobic conditions, oxygen or oxygenous gas mixtures such as, for example, air are introduced into the culture.

The temperature of the culture is normally from 25°C to 45°C and preferably from 30°C to 40°C. The activity of the microorganisms leads to an enrichment or accumulation of L-tryptophan in the fermentation or culture broth. The culture is continued until a maximum of L-tryptophan has formed. This target is normally reached within 10 hours to 160 hours. In continuous processes, longer cultivation times are possible.

A fermentation broth or culture broth is understood to mean a fermentation medium in which a microorganism has been cultivated for a certain time and at a certain temperature. On completion of the fermentation, the resulting fermentation broth accordingly comprises a) the biomass (= cell mass) of the microorganism that arises as a result of the propagation of the cells of the microorganism, b) the L-amino acid (L-tryptophan) formed during the fermentation, c) the organic by-products formed during the fermentation and d) the constituents of the fermentation medium/media used or of the starting materials that are not consumed by the fermentation, such as, for example, vitamins like thiamine or salts like magnesium sulphate.

The culture broth or fermentation broth produced can then be collected and the L-tryptophan or the L-tryptophan -containing product can be obtained or isolated. In one method variant, the fermentation broth is concentrated if necessary and then the L-tryptophan is purified or is isolated in a pure or virtually pure form. Typical methods for purifying L-amino acids such as L-tryptophan are ion-exchange chromatography, crystallization, extraction methods and treatment with activated carbon. What are obtained as a result are largely pure L-tryptophan having a content of ≥ 90% by weight, ≥ 95% by weight, ≥ 96% by weight, ≥ 97% by weight, ≥ 98% by weight or ≥ 99% by weight.

In another method variant, it is likewise possible to produce a product from the fermentation broth produced, by removing the bacterium biomass present in the fermentation broth to a complete extent (100%) or to a virtually complete extent, i.e. more than or greater than (>) 90%, >95%, >97%, >99%, and by leaving in the product the remaining constituents of the fermentation broth to a large extent, i.e. to an extent of 30% - 100%, 40% - 100%, 50% - 100%, 60% - 100%, 70% - 100%, 80% - 100%, or 90% - 100%, preferably greater than or equal to (≥) 50%, ≥60%, ≥70%, ≥80%, ≥90% or ≥95%, or else to a complete extent (100%).

The biomass is removed or separated off by using separation methods such as, for example, centrifugation, filtration, decanting, flocculation or a combination thereof. The broth obtained is then thickened or concentrated using known methods such as, for example, with the aid of a rotary evaporator, thin-film evaporator, falling-film evaporator, by reverse osmosis, by nanofiltration or a combination thereof.

This concentrated broth is then worked up by methods of freeze drying, of spray drying, of spray granulation or by other processes to give a preferably free-flowing, fine powder. This free-flowing, fine powder can then in turn be converted by suitable compaction or granulation processes into a coarse, highly free-flowing, storable and substantially dust-free product. Here, the water is altogether removed to an extent of more than 90%, and so the water content in the product is less than 10% by weight, less than 5% by weight, less than 4% by weight or less than 3% by weight.

The analysis of L-tryptophan to determine the concentration at one or more times during the fermentation can be effected by separating the L-tryptophan by means of ion-exchange chromatography, preferably cation-exchange chromatography, with subsequent post-column derivatization using ninhydrin, as described in Spackman et al. (Analytical Chemistry 30: 1190-1206 (1958)). It is also possible to employ ortho-phthalaldehyde rather than ninhydrin for post-column derivatization. An overview article on ion-exchange chromatography can be found in Pickering (LC·GC (Magazine of Chromatographic Science) 7(6), 484-487 (1989)).

It is likewise possible to carry out a pre-column derivatization, for example using ortho-phthalaldehyde or phenyl isothiocyanate, and to fractionate the resulting amino acid derivatives by reversed-phase chromatography (RP), preferably in the form of high-performance liquid chromatography (HPLC). Such a method is, for example, described in Lindroth et al. (Analytical Chemistry 51: 1167-1174 (1979)). Detection is carried out photometrically (absorption, fluorescence).

The process and the microorganism according to the invention is used for fermentatively preparing L-tryptophan.

### Brief description of the figures

- Figure 1:: Map of the DaroP deletion fragment-containing replacement vector pKO3DaroP
- Figure 2:: Map of the mdfA gene-containing replacement vector pKO3DaroP::mdfA
- Figure 3:: Map of the Dmtr deletion fragment-containing replacement vector pKO3Dmtr
- Figure 4:: Map of the mdfA gene-containing replacement vector pKO3Dmtr::mdfA
- Figure 5:: Map of the plasmid pMU91

Specified lengths are to be understood as approximations. The meanings of the abbreviations and designations used are as follows:

| | |
|---|---|
| • CmR: | Chloramphenicol-resistance gene |
| • sacB: | sacB gene from Bacillus subtilis |
| • RepA: | Temperature-sensitive replication region of the plasmid pSC101 |
| • pdhR': | Part of the coding region of the pdhR gene |
| • mdfA: | Coding region of the mdfA gene |
| • 'ampE: | Part of the coding region of the ampE gene |
| • 'yhbW: | Part of the coding region of the yhbW gene |
| • 'deaD: | Part of the coding region of the deaD gene |
| • pSC101: | Plasmid fragment pSC101 |
| • serA: | Coding region of the serA gene encoding D-3-phosphoglycerate dehydrogenase |
| • trpE476DCBA: | Part of the tryptophan operon trpLEDCBA with trpE476 allele |
| • tetA: | Tetracycline-resistance gene |

The meanings of the abbreviations for the restriction enzymes are as follows
- SalI: Restriction endonuclease from Streptomyces albus G
- HindIII: Restriction endonuclease from Haemophilus influenzae Rd
- XhoI: Restriction endonuclease from Xanthomonas holcicola

Further details may be found in the examples.

In the following, the invention is illustrated by non-limiting examples and exemplifying embodiments.

### Examples

Minimal (M9) and full media (LB) used for Escherichia coli are described by J.H. Miller (A Short Course in Bacterial Genetics (1992), Cold Spring Harbor Laboratory Press). The isolation of plasmid DNA from Escherichia coli and all techniques for restriction digestion, ligation, Klenow treatment and alkaline-phosphatase treatment are carried out as per Sambrook et al. (Molecular Cloning - A Laboratory Manual (1989) Cold Spring Harbor Laboratory Press). Unless otherwise described, the transformation of Escherichia coli is carried out in accordance with Chung et al. (Proceedings of the National Academy of Sciences of the United States of America, USA (1989) 86: 2172-2175).

Unless otherwise described, the incubation temperature in the production of strains and transformants is 37°C.

### Example 1

### Construction of the replacement vector pKO3DaroP

The deletion flanks for the deletion of aroP are amplified using the polymerase chain reaction (PCR) and the deletion fragment was produced by overlap PCR. Proceeding from the nucleotide sequence of the aroP gene in E. coli K-12 MG1655 (accession number NC_000913.3, region: 120178-121551, Blattner et al. (Science 277: 1453-1462 (1997)) and the flanking regions, PCR primers are synthesized (Eurofins Genomics GmbH (Ebersberg, Germany)). The primers are designed such that the entire aroP gene is deleted.

### Primer design and PCR of the two flanks

### Flank 1

| | |
|---|---|
| **aroP-up1** | 5' GATCTGAGCTCTAGACCTGGCGACGAAGCAACAAG 3' XbaI SEQ ID NO.: 3 |
| **aroP-up6** | 5'        GCGTTGGTGTAATCGCGAACCTCGTGCGGTGGTTGTT        3' NruI                                    SEQ ID NO.: 4 |

The chromosomal E. coli MG1655 DNA used for the PCR is isolated using the QIAGEN DNeasy Blood & Tissue Kit (QIAGEN GmbH, Hilden, Germany) according to the information from the manufacturer. Using the two specific primers "aroP-up1" and "aroP-up6", PCR amplification is carried out under standard PCR conditions (Innis et al.: PCR Protocols. A Guide to Methods and Applications, 1990, Academic Press) using the Phusion DNA Polymerase (Thermo Fisher Scientific, Wattham, MA USA) with MG1655 DNA as template to amplify the fragment "aroP-Flank1" (length: 775 bp). Via the primers, an Xbal and an Nrul restriction site are inserted into the PCR product.

### Flank 2

| | |
|---|---|
| **aroP-down2** | 5'     GATCTATCTAGACTGTTACGCGCATTGCAGG   3' XbaI     SEQ ID NO.: 5 |
| **aroP-down3** | 5' ACCGCACGAGGTTCGCGATTACACCAACGCCCCGTAAATCG 3' NruI SEQ ID NO.: 6 |

Using the two primers "aroP-down2" and "aroP-down3", PCR is carried out with MG1655 DNA as template to amplify the fragment "aroP-Flank2" (length: 717 bp). Via the primers, an Xbal and an Nrul restriction site are inserted into the PCR product.

### Fusion of the two flanks by overlap PCR

The two flanks are fused together via their overlapping regions by means of an overlap PCR using the two outer primers "aroP-up1" and "aroP-down2". The resulting product "aroP-Del-Fragment" has a length of 1462 bp. Recognition sequences for the restriction enzyme Xbal are at both ends.

### Cloning of the insert into pKO3

The above fusion product is purified using the Qiaquick PCR Purification Kit (QIAGEN GmbH, Hilden, Germany) and then digested using Xbal. This generates Xbal "sticky ends". The digest is purified again using the Qiaquick PCR Purification Kit, and this also results in the short cut-off sequences being removed.

The vector pKO3 is likewise cut using Xbal and simultaneously dephosphorylated using alkaline phosphatase. Thereafter, the digest is purified using the Qiaquick PCR Purification Kit, and this also results in the short fragment between the Xbal sites being removed. The restriction product likewise has Xbal "sticky ends". These are unphosphorylated, preventing a self-ligation of the plasmid.

Ligation is carried out by using T4 ligase to ligate vector and insert in the molar ratio of 1:3. Chemically competent cells of the *E. coli* cloning strain NEB5alpha are transformed using 1 µl of the ligation reaction and spread out on LB agar containing 20 mg/l chloramphenicol. The plates are incubated at 30°C for 40 h.

### Checking of the plasmid clones

Successful cloning is verified by cleavage of the plasmid pKO3DaroP using the restriction enzyme Aval.

10 colonies are picked and cultivated overnight at 30°C/180 rpm in 10 ml LB+20 mg/l chloramphenicol in each case.

The following day, 2 ml of the cultures are centrifuged in each case and minipreps are prepared from the pellets.

The ligation products can contain the insert in two orientations. An Aval restriction allows to prove the insert as well as to determine the orientation:
- Insert in orientation s: Fragments 148 bp, 1447 bp and 5494 bp
- Insert in orientation as: Fragments 1351 bp, 1447 bp and 4291 bp
- pKO3 empty vector: Fragments 1043 bp and 5263 bp

The 10 plasmid clones are cut using Aval and the products are separated on a 0.8% TAE agarose gel. One plasmid clone containing the insert in orientation "s" was selected and called "pKO3DaroP".

The insert of said clone is sequenced using the primers "pKO3-L" and "pKO3-R".

| | |
|---|---|
| **pKO3-L** 5' AGGGCAGGGTCGTTAAATAGC 3' | SEQ ID NO.: 7 |
| **pKO3-R** 5' TTAATGCGCCGCTACAGGGCG 3' | SEQ ID NO.: 8 |

The DNA sequence of the amplified fragment "DaroP" is determined by using the primers "pKO3-L" and "pKO3-R" (Eurofins Genomics GmbH (Ebersberg, Germany)). The expected sequence of the fragment was confirmed and the cloned fragment is described in SEQ ID NO.:9.

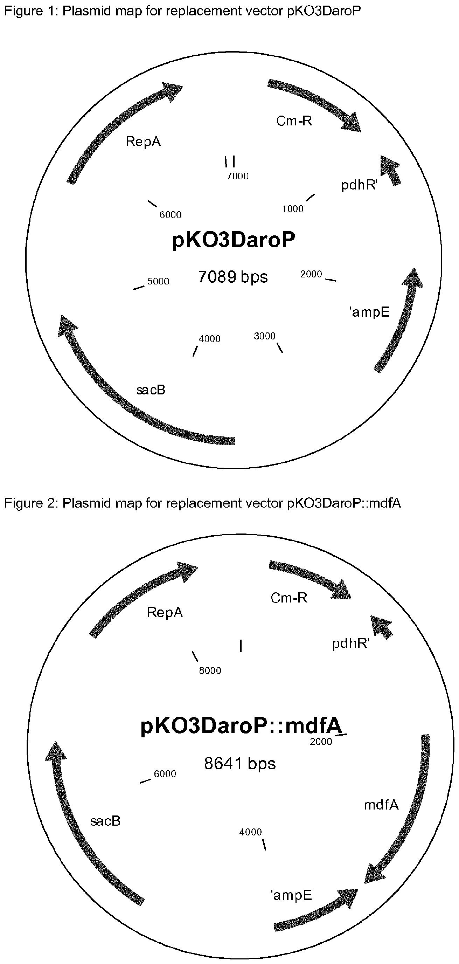

The resultant replacement vector pKO3DaroP is depicted in Figure 1.

### Example 2

### Construction of the replacement vector pKO3Dmtr

The deletion flanks for the deletion of mtr are amplified using the polymerase chain reaction (PCR) and the deletion fragment was produced by overlap PCR. Proceeding from the nucleotide sequence of the mtr gene in E. coli K-12 MG1655 (accession number NC_000913.3, region: 3304573-3305817, Blattner et al. (Science 277: 1453-1462 (1997)) and the flanking regions, PCR primers are synthesized (Eurofins Genomics GmbH (Ebersberg, Germany)). The primers are designed such that the entire mtr gene is deleted.

### Primer design and PCR of the two flanks

### Flank 1

| | | |
|---|---|---|
| **mtr-del-Xba-1** | 5' | TTGAGAACCGCGAGCGTCGTCTG 3' SEQ ID NO.:10 |
| **mtr-del-Xba-2** | 5' | TCTAGATTCGCGAGGGCTTCTCTCCAGTGAAA 3' XbaI NruI SEQ ID NO.: 11 |

The chromosomal E. coli MG1655 DNA used for the PCR is isolated using the QIAGEN DNeasy Blood & Tissue Kit (QIAGEN GmbH, Hilden, Germany) according to the information from the manufacturer. Using the two specific primers "mtr-del-Xba-1" and "mtr-del-Xba-2", PCR is carried out under standard PCR conditions (Innis et al.: PCR Protocols. A Guide to Methods and Applications, 1990, Academic Press) using the Phusion DNA Polymerase (Thermo Fisher Scientific, Wattham, MA USA) with MG1655 DNA as template to amplify the fragment "mtr-Flank1" (length: 1009 bp). Via the primers, an Xbal and an Nrul restriction site are inserted into the PCR product.

### Flank 2

| | | |
|---|---|---|
| **mtr-del-Xba-3** | 5' | AAGCCCTCGCGAATCTAGAGTAATCAGCGGTGCCTTATC 3' NruI XbaI SEQ ID NO.: 12 |
| **mtr-del-Xba-4** | 5' | GCTGGCAGAACACCACAATATG 3' SEQ ID NO.: 13 |

Using the two primers "mtr-del-Xba-3" and "mtr-del-Xba-4", PCR is carried out with MG1655 DNA as template to amplify the fragment "mtr-Flank2" (length: 1014 bp). Via the primers, an Xbal and an Nrul restriction site are inserted into the PCR product.

### Fusion of the two flanks by overlap PCR

The two flanks are fused together via their overlapping regions by means of an overlap PCR using the two outer primers "mtr-del-Xba-1" and "mtr-del-Xba-4". The resulting product "mtr-del-Fragment" has a length of 2004 bp.

### Cloning of the insert into pKO3

According to information from the manufacturer, the amplified "mtr-del-Fragment" is ligated into the vector pCR-Blunt II-TOPO (Zero Blunt TOPO PCR Cloning Kit, Thermo Fisher Scientific, Wattham, MA USA) and transformed into E. coli TOP10. Cells containing a plasmid are selected on LB agar containing 50 µg/ml kanamycin. After isolation of the plasmid DNA, successful cloning is verified by cleavage of the plasmid using the restriction enzyme HincII and separation of the products on a 0.8% TAE agarose gel. The successfully cloned vector is called "pCRBI-mtr-del".

The vector is then cleaved using the restriction enzymes NotI and SpeI and the mtr-del-Fragment is resolved on a 0.8% TAE agarose gel. This is followed by the isolation of the fragment from the gel using the QIAquick Gel Extraction Kit (QIAGEN GmbH, Hilden, Germany) and cloning into the replacement vector pKO3 (Link et al, 1997, J. Bacteriol., 179, 20, 6228-6237).

The vector pKO3 is cut using NotI and XbaI. Thereafter, the digest is purified using the Qiaquick PCR Purification Kit (QIAGEN GmbH, Hilden, Germany).

Ligation is carried out by using T4 ligase to ligate vector and insert in the molar ratio of 1:3. Chemically competent cells of the *E. coli* cloning strain NEB5alpha are transformed using 1 µl of the ligation reaction and spread out on LB agar containing 20 mg/l chloramphenicol. The plates are incubated at 30°C for 40 h.

### Checking of the plasmid clones

Successful cloning is verified by cleavage of the plasmid pKO3Dmtr using the restriction enzyme HincII.

Correct clones have the following cleavage pattern:
- Insert mtr-del: Fragments 822 bp, 1251 bp, 1611 bp and 4020 bp
- pKO3 empty vector: Fragments 822 bp, 1617 bp and 3232 bp
10 colonies are picked and cultivated overnight at 30°C/180 rpm in 10 ml LB+20 mg/l chloramphenicol in each case.

The following day, 2 ml of the cultures are centrifuged in each case and minipreps are prepared from the pellets.

The 10 plasmid clones are cut using HincII and the products are separated on a 0.8% TAE agarose gel. One plasmid clone containing the correct insert was selected and called "pKO3Dmtr".

The insert of said clone is sequenced using the primers "pKO3-L" and "pKO3-R".

| | |
|---|---|
| **pKO3-L** 5' AGGGCAGGGTCGTTAAATAGC 3' | SEQ ID NO.: 7 |
| **pKO3-R** 5' TTAATGCGCCGCTACAGGGCG 3' | SEQ ID NO.: 8 |

The DNA sequence of the amplified fragment "Dmtr" is determined by using the primers "pKO3-L" and "pKO3-R" (Eurofins Genomics GmbH (Ebersberg, Germany)). The expected sequence of the fragment was confirmed and the cloned fragment is described in SEQ ID NO.: 14.

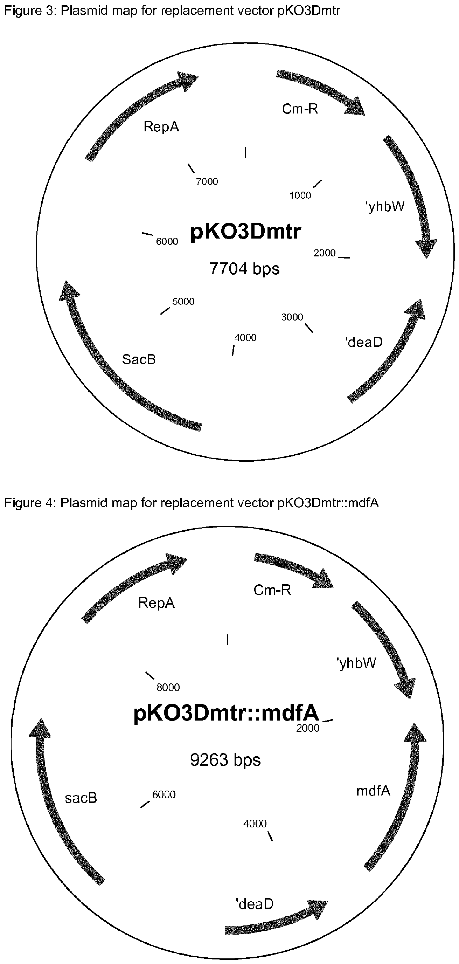

The resultant replacement vector pKO3Dmtr is depicted in Figure 3.

### Example 3

### Construction of the replacement vectors pKO3DaroP::mdfA and pKO3Dmtr::mdfA

### 3.1 Construction of the vector pB-mdfA

The mdfA allele is amplified using the polymerase chain reaction (PCR). Proceeding from the nucleotide sequence of the mdfA gene in E. coli K12 MG1655 (accession number NC_000913.3, region: 883673-884905, Blattner et al. (Science 277: 1453-1462 (1997)), PCR primers are synthesized (Eurofins Genomics GmbH (Ebersberg, Germany)).

### Primer design and PCR of the mdfA gene region

| | | |
|---|---|---|
| **cmr-for:** | 5' | CGTCGCGATACAGGCAAGTCGTTGAG 3' SEQ ID NO.: 15 NruI |
| **cmr-rev:** | 5' | CCTCGCGACAGATTGACGACCATCAC 3' SEQ ID NO.: 16 NruI |

The chromosomal E. coli MG1655 DNA used for the PCR is isolated using the QIAGEN DNeasy Blood & Tissue Kit (QIAGEN GmbH, Hilden, Germany) according to the information from the manufacturer. Using the two specific primers "crmr-for" and "cmr-rev", PCR is carried out under standard PCR conditions (Innis et al.: PCR Protocols. A Guide to Methods and Applications, 1990, Academic Press) using the Phusion DNA Polymerase (Thermo Fisher Scientific, Wattham, MA USA) with MG1655 DNA as template to amplify the fragment "mdfA-Insert" (length: 1562 bp), described in SEQ ID NO.: 19. Via the primers, Nrul restriction sites are inserted into the PCR product.

### Cloning of the insert into pCR-Blunt II

According to information from the manufacturer, the amplified fragment "mdfA-Insert" is ligated into the vector pCR-Blunt II-TOPO (Zero Blunt TOPO PCR Cloning Kit, Thermo Fisher Scientific, Wattham, MA USA) and transformed into E. coli TOP10. Cells containing a plasmid are selected on LB agar containing 50 µg/ml kanamycin. After isolation of the plasmid DNA, successful cloning is verified by cleavage of the plasmid using the restriction enzyme Sphl and separation of the products on a 0.8% TAE agarose gel.

The ligation products can contain the insert in two orientations. A Sphl restriction allows to prove the insert as well as to determine the orientation:
- Insert in orientation s: Fragments 1351 bp, 1385 bp and 2345 bp
- Insert in orientation as: Fragments 305 bp, 1385 bp and 3391 bp
- pCR-Blunt II empty vector: Fragments 1385 bp and 2134 bp

The plasmid clones are cut using Sphl and the products are separated on a 0.8% TAE agarose gel. One plasmid clone containing the insert in orientation "as" was selected and called "pB-mdfA".

The insert of said clone is sequenced using the primers "M13uni(-21)" and "M13rev(-29)".

| | |
|---|---|
| **M13uni(-21)** 5' TGTAAAACGACGGCCAGT 3' | SEQ ID NO.: 17 |
| **M13rev(-29)** 5' CAGGAAACAGCTATGACC 3' | SEQ ID NO.: 18 |

The DNA sequence of the amplified fragment "mdfA" is determined by using the primers "M13uni(-21)" and "M13rev(-29)" (Eurofins Genomics GmbH (Ebersberg, Germany)). The expected sequence of the fragment was confirmed.

### 3.2 Construction of the vector pKO3DaroP::mdfA

The vector pB-mdfA is cleaved using the restriction enzyme NruI and the mdfA fragment is resolved on a 0.8% TAE agarose gel. This is followed by the isolation of the fragment from the gel using the QIAquick Gel Extraction Kit (QIAGEN GmbH, Hilden, Germany) and cloning into the replacement vector pKO3DaroP that was generated.

The vector pKO3DaroP is cut using NruI and simultaneously dephosphorylated using alkaline phosphatase. This cleavage preserves the promoter region of aroP that is still present. Thereafter, the digest is purified using the Qiaquick PCR Purification Kit (QIAGEN GmbH, Hilden, Germany).

Ligation is carried out by using T4 ligase to ligate vector and insert in the molar ratio of 1:3. Chemically competent cells of the *E. coli* cloning strain NEB5alpha are transformed using 1 µl of the ligation reaction and spread out on LB agar containing 20 mg/l chloramphenicol. The plates are incubated at 30°C for 40 h.

### Checking of the plasmid clones

Successful cloning is verified by cleavage of the plasmid pKO3DaroP::mdfA using the restriction enzyme Asel.

10 colonies are picked and cultivated overnight at 30°C/180 rpm in 10 ml LB+20 mg/l chloramphenicol in each case.

The following day, 2 ml of the cultures are centrifuged in each case and minipreps are prepared from the pellets.

The ligation products can contain the insert in two orientations. An Asel restriction allows to prove the insert as well as to determine the orientation:
- Insert in orientation s: Fragments 1603 bp and 7038 bp
- Insert in orientation as: Fragments 101 bp and 8540 bp

The 10 plasmid clones are cut using Asel and the products are separated on a 0.8% TAE agarose gel. One plasmid clone containing the insert in orientation "s" was selected and called "pKO3DaroP::mdfA".

The insert of said clone is sequenced using the primers "pKO3-L", "pKO3-R", "cmr-for" and "cmr-rev".

| | | |
|---|---|---|
| **pKO3-L** | 5' AGGGCAGGGTCGTTAAATAGC 3' | SEQ ID NO.:7 |
| **pKO3-R** | 5' TTAATGCGCCGCTACAGGGCG 3' | SEQ ID NO.:8 |
| **cmr-for** | 5' TACAGGCAAGTCGTTGAG 3' | SEQ ID NO.:15 |
| **cmr-rev** | 5' CAGATTGACGACCATCAC 3' | SEQ ID NO.:16 |

The DNA sequence of the insert "DaroP::mdfA" is determined by using the primers "pKO3-L", "pKO3-R", "cmr-for" and "cmr-rev" (Eurofins Genomics GmbH (Ebersberg, Germany)). The expected sequence of the fragment was confirmed and the cloned fragment is described in SEQ ID NO.: 20.

The resultant replacement vector pKO3DaroP::mdfA is depicted in Figure 2.

### 3.3 Construction of the vector pKO3Dmtr::mdfA

The vector pB-mdfA is cleaved using the restriction enzyme Nrul and the mdfA fragment is resolved on a 0.8% TAE agarose gel. This is followed by the isolation of the fragment from the gel using the QIAquick Gel Extraction Kit (QIAGEN GmbH, Hilden, Germany) and cloning into the replacement vector pKO3Dmtr that was generated.

The vector pKO3Dmtr is cut using NruI and simultaneously dephosphorylated using alkaline phosphatase. This cleavage preserves the promoter region of mtr that is still present. Thereafter, the digest is purified using the Qiaquick PCR Purification Kit (QIAGEN GmbH, Hilden, Germany).

Ligation is carried out by using T4 ligase to ligate vector and insert in the molar ratio of 1:3. Chemically competent cells of the *E. coli* cloning strain NEB5alpha are transformed using 1 µl of the ligation reaction and spread out on LB agar containing 20 mg/l chloramphenicol. The plates are incubated at 30°C for 40 h.

### Checking of the plasmid clones

Successful cloning is verified by cleavage of the plasmid pKO3Dmtr::mdfA using the restriction enzyme HincII.

10 colonies are picked and cultivated overnight at 30°C/180 rpm in 10 ml LB+20 mg/l chloramphenicol in each case.

The following day, 2 ml of the cultures are centrifuged in each case and minipreps are prepared from the pellets.

The ligation products can contain the insert in two orientations. A HincII restriction allows to prove the insert as well as to determine the orientation:
- Insert in orientation s: Fragments 354 bp, 822 bp, 1613 bp, 2449 bp and 4025 bp
- Insert in orientation as: Fragments 822 bp, 1181 bp, 1613 bp, 1622 bp and 4025 bp

The 10 plasmid clones are cut using HincII and the products are separated on a 0.8% TAE agarose gel. One plasmid clone containing the insert in orientation "s" was selected and called "pKO3Dmtr::mdfA".

The insert of said clone is sequenced using the primers "pKO3-L", "pKO3-R", "cmr-for" and "cmr-rev".

| | | |
|---|---|---|
| **pKO3-L** | 5' AGGGCAGGGTCGTTAAATAGC 3' | SEQ ID NO.:7 |
| **pKO3-R** | 5' TTAATGCGCCGCTACAGGGCG 3' | SEQ ID NO.:8 |
| **cmr-for** | 5' TACAGGCAAGTCGTTGAG 3' | SEQ ID NO.:15 |
| **cmr-rev** | 5' CAGATTGACGACCATCAC 3' | SEQ ID NO.:16 |

The DNA sequence of the insert "Dmtr::mdfA" is determined by using the primers "pKO3-L", "pKO3-R", "cmr-for" and "cmr-rev" (Eurofins Genomics GmbH (Ebersberg, Germany)). The expected sequence of the fragment was confirmed and the cloned fragment is described in SEQ ID NO.: 21.

The resultant replacement vector pKO3Dmtr::mdfA is depicted in Figure 4.

### Example 4

### Replacement of the aroP allele of strain DM2186 by the deletion fragments DaroP and DaroP::mdfA

The L-tryptophan-producing E. coli strain DM2186/pMU91 is a P1-transduction-obtainable trpS⁺ derivative of the Escherichia coli K-12 strain JP6015/pMU91 described in the patent specification US-A-5,756,345. pMU91 is a plasmid derived from pSC101 (Cohen et al., Journal of Bacteriology 132: 734-737 (1977)), which bears Tet^{R}, trpE476DCBA and serA⁺. JP6015/pMU91 is deposited as DSM 10123 at the Leibniz Institute DSMZ - German Collection of Microorganisms and Cell Cultures (DSMZ, Braunschweig, Germany) in accordance with the Budapest treaty. DM2186 is deposited on November 22, 2018 as DSM 32961 at the Leibniz Institute DSMZ - German Collection of Microorganisms and Cell Cultures (DSMZ, Braunschweig, Germany) in accordance with the Budapest treaty.

In the chromosomal aroP allele of the strain DM2186, there is a frame-shift mutation.

The replacement of the chromosomal aroP allele by the plasmid-coded deletion construct was carried out by transforming DM2186 with the plasmid pKO3DaroP. The gene replacement is done using the selection method described by Link et al. (Journal of Bacteriology 179: 6228-6237 (1997)) and was verified by sequencing.

After replacement has been carried out, what is present in DM2186 is the form of the deleted aroP allele that is described in SEQ ID NO.:22 (sequencing by Eurofins Genomics GmbH (Ebersberg, Germany)). The strain obtained is referred to as DM2186DaroP.

Increased expression of the mdfA gene in the E. coli strain DM2186/pMU91 integrated in the aroP locus

The replacement of the chromosomal aroP allele by the plasmid-coded mdfA allele with simultaneous deletion of the aroP gene, the promoter region of aroP being preserved, was carried out by transforming DM2186 with the plasmid pKO3DaroP::mdfA. The gene replacement is done using the selection method described by Link et al. (Journal of Bacteriology 179: 6228-6237 (1997)) and was verified by sequencing.

After replacement has been carried out, what is present in DM2186 is the form of the mdfA gene, as described in SEQ ID NO.: 23, integrated in the aroP locus with simultaneous deletion of the aroP allele (sequencing by Eurofins Genomics GmbH (Ebersberg, Germany)). The strain obtained is referred to as DM2186DaroP::mdfA.

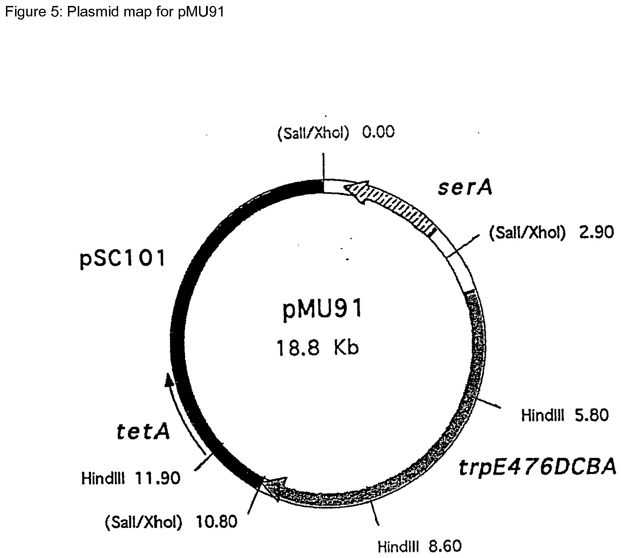

The plasmid pMU91 which is described in the patent specification US-A-5,756,345 and which carries the genetic information for tryptophan production was isolated from the strain JP6015/pMU91. The plasmid is depicted in Figure 5. The strains DM2186DaroP and DM2186DaroP::mdfA were each transformed with this plasmid. The respective transformants are referred to as DM2186DaroP/pMU91 and DM2186DaroP::mdfA/pMU91.

### Example 5

### Replacement of the mtr allele of strain DM2186 by the deletion fragments Dmtr and Dmtr::mdfA

The L-tryptophan-producing E. coli strain DM2186/pMU91 is a P1-transduction-obtainable trpS⁺ derivative of the Escherichia coli K-12 strain JP6015/pMU91 described in the patent specification US-A-5,756,345. pMU91 is a plasmid derived from pSC101 (Cohen et al., Journal of Bacteriology 132: 734-737 (1977)), which bears Tet^{R}, trpE476DCBA and serA⁺. JP6015/pMU91 is deposited as DSM 10123 at the Leibniz Institute DSMZ - German Collection of Microorganisms and Cell Cultures (DSMZ, Braunschweig, Germany) in accordance with the Budapest treaty. DM2186 is deposited on November 22, 2018 as DSM 32961 at the Leibniz Institute DSMZ - German Collection of Microorganisms and Cell Cultures (DSMZ, Braunschweig, Germany) in accordance with the Budapest treaty.

In the chromosomal mtr allele of the strain DM2186, there is a frame-shift mutation.

The replacement of the chromosomal mtr allele by the plasmid-coded deletion construct was carried out by transforming DM2186 with the plasmid pKO3Dmtr. The gene replacement is done using the selection method described by Link et al. (Journal of Bacteriology 179: 6228-6237 (1997)) and was verified by sequencing.

After replacement has been carried out, what is present in DM2186 is the form of the deleted mtr allele that is described in SEQ ID NO.: 24 (sequencing by Eurofins Genomics GmbH (Ebersberg, Germany)). The strain obtained is referred to as DM2186Dmtr.

Increased expression of the mdfA gene in the E. coli strain DM2186/pMU91 integrated in the mtr locus

The replacement of the chromosomal mtr allele by the plasmid-coded mdfA allele with simultaneous deletion of the mtr gene, the promoter region of mtr being preserved, was carried out by transforming DM2186 with the plasmid pKO3Dmtr::mdfA. The gene replacement is done using the selection method described by Link et al. (Journal of Bacteriology 179: 6228-6237 (1997)) and was verified by sequencing.

After replacement has been carried out, what is present in DM2186 is the form of the mdfA gene, as described in SEQ ID NO.: 25, integrated in the mtr locus with simultaneous deletion of the mtr allele (sequencing by Eurofins Genomics GmbH (Ebersberg, Germany)). The strain obtained is referred to as DM2186Dmtr::mdfA.

The plasmid pMU91 which is described in the patent specification US-A-5,756,345 and which carries the genetic information for tryptophan production was isolated from the strain JP6015/pMU91. The plasmid is depicted in Figure 5. The strains DM2186Dmtr and DM2186Dmtr::mdfA were each transformed with this plasmid. The respective transformants are referred to as DM2186Dmtr/pMU91 and DM2186Dmtr::mdfA/pMU91.

### Example 6

### Production of L-tryptophan using the strains DM2186DaroP/pMU91, DM2186DaroP::mdfA/pMU91, DM2186Dmtr/pMU91 and DM2186Dmtr::mdfA/pMU91

To test the effect of an additional copy of the mdfA gene expressed from various regulatory sequences in two different chromosomal environments DM2186DaroP/pMU91, DM2186DaroP::mdfA/pMU91, DM2186Dmtr/pMU91, DM2186Dmtr::mdfA/pMU91 and DM2186/pMU91 were further propagated at 30°C on LB medium having the following composition: 10 g/l Bacto tryptone, 5 g/l yeast extract, 10 g/l NaCl (pH adjustment to 7.5 using NaOH), 2 g/l glucose, 20 g/l agar and 5 mg/l tetracycline. The formation of L-tryptophan is checked in batch cultures of 10 ml contained in 100 ml conical flasks. To this end, 10 ml of preculture medium of the following composition are inoculated, and incubated at 30°C and 180 rpm for 16 hours on an Infors HT Multitron standard incubator from Infers AG (Bottmingen, Switzerland): 1 g/l yeast extract, 100 ml/l MOPS buffer (10x), 10 g/l glucose, 0.1 mg/l thiamine and 5 mg/l tetracycline.

10x MOPS buffer is prepared from solutions A and B according to Tables 1 to 3; two volumes of solution A are added aseptically to three volumes of solution B.

**Table 1: Solution A for 10x MOPS buffer (sterile-filtered).**

| Component | Concentration |
|---|---|
| MOPS (morpholinopropanesulfonic acid) | 418.6 g/L |
| KOH (solid) | For adjustment of pH to 7.4 |

**Table 2: Solution B for 10x MOPS buffer. Sterilized by autoclaving (30 minutes, 121°C).**

| Component | Concentration |
|---|---|
| Na₃ citrate x 2H₂O | 2.35 g/l |
| FeSO₄ x 7H₂O | 0.22 g/l |
| NH₄Cl | 32.0 g/l |
| MgSO₄ x 7H₂O | 6.7 g/l |
| KCl | 4.0 g/l |
| CaCl₂ x 2H₂O | 0.25 mg/l |
| Trace-elements stock solution (Tab. 4) | 3.33 ml/l |

**Table 3: Trace-elements stock solution (in demin. water) for 10x MOPS buffer.**

| Component | Concentration |
|---|---|
| (NH₄)₆Mo₇O₂₄ x 4H₂O | 3.7 mg/l |
| H₃BO₃ | 24.0 mg/l |
| CoCl₂ x 6H₂O | 7.1 mg/l |
| ZnSO₄ x 7H₂O | 28.7 mg/l |
| MnCl₂ x 4H₂O | 15.8 mg/l |
| CuSO₄ x 5H₂O | 2.5 mg/l |

100 µl of said preculture are in each case inoculated in 10 ml of production medium PM3P according to Table 4 and incubated at 33°C and 180 rpm for 44 hours on an Infers HT Multitron standard incubator from Infers AG (Bottmingen, Switzerland). After incubation, the optical density (OD) of the culture suspension is determined using a Nanocolor 400D photometer from Macherey-Nagel GmbH & Co. KG (Düren, Germany) at a measurement wavelength of 660 nm.

**Table 4: PM3P medium: for production tests in 100 ml conical flasks**

| Component | Concentration |
|---|---|
| 10x MOPS buffer | 100 ml/l |
| Thiamine HCl (0.01%) | 1.0 ml/l |
| KH2PO4 (45 mM) | 10 ml/l |
| Glucose | 10 g/l |
| Tyrosine | 0.02 g/l |
| Phenylalanine | 0.02 g/l |
| Tetracycline | 5 mg/l |

Thereafter, the concentration of L-tryptophan formed in the sterile-filtered culture supernatant is determined by reversed-phase HPLC, for instance as described in Lindroth et al. (Analytical Chemistry (1979) 51: 1167-1174).

Table 5 shows the result of the experiment.

**Table 5: Production of L-tryptophan using the strains DM2186DaroP/pMU91, DM2186DaroP::mdfA/pMU91, DM2186Dmtr/pMU91 and DM2186Dmtr::mdfA/pMU91**

| Strain | OD (660 nm) | L-Tryptophan, g/l | Yield, net % |
|---|---|---|---|
| DM2186DaroP/pMU91 | 4.17 | 1.63 | 13.60 |
| DM2186DaroP::mdfA/pMU91 | 4.02 | 1.66 | 13.82 |
| DM2186Dmtr/pMU91 | 4.29 | 1.62 | 13.61 |
| DM2186Dmtr::mdfA/pMU91 | 3.85 | 1.82 | 15.16 |
| DM2186/pMU91 | 4.38 | 1.62 | 13.54 |

As derivable therefrom, the enhancement of mdfA gene increases the yield of L-tryptophan. However, the expression level of mdfA - and thereby the L-tryptophan yield - is strongly influenced by choosing the integration locus and the chromosomal environment of the integrated mdfA gene or allele.

Integration of the mdfA into the chromosomal aroP locus leads to a slight increase in L-tryptophan productivity, whereas integration of the mdfA into the chromosomal mtr locus leads to a strong increase in L-tryptophan productivity, i.e. the chromosomal environment regulates the expression of mdfA. The positive effect on L-tryptophan synthesis is much more pronounced in DM2186Dmtr::mdfA/pMU91 compared to the integration of mdfA into the aroP gene locus. Obviously, creation of a defined expression level of the additional copy of the mdfA gene in a specific gene locus is more important for L-tryptophan production than the general enhancement of expression by gene copy number increasement. Especially the expression levels of membrane proteins, such as transport proteins and amino acid exporters have to be modulated in an appropriate way without affecting cellular fitness and production capacities when commercial relevant concentrations accumulate in the fermentation broth.

## Claims

1. Method of producing L-tryptophan, the method comprising culturing a L-tryptophan producing microorganism belonging to the *Enterobacteriaceae* family in a fermentation medium;
the method being **characterized in that** the L-tryptophan producing microorganism has been modified by enhancing the expression level of the mdfA gene or by enhancing the expression level of an mdfA allele.

2. Method according to claim 1, the method being **characterized in that** the mdfA allele is a polynucleotide selected from the group consisting of:
a) a polynucleotide sequence comprising the nucleotide sequence of SEQ ID NO.: 1;
b) a polynucleotide that hybridizes with a complementary strand of SEQ ID NO.: 1 under stringent conditions;
c) a naturally occurring mutant or polymorphic form of a polynucleotide according to a) or b);
d) a polynucleotide having a sequence identity of at least 80% of the nucleotide sequence of SEQ ID NO.: 1;
e) a polynucleotide including substitutions, deletions, insertions or additions of 1 to 60 nucleotides in relation to the nucleotide sequence of SEQ ID NO.: 1;
f) a polynucleotide encoding a protein comprising the amino acid sequence of SEQ ID NO.: 2;
g) a polynucleotide encoding a protein comprising an amino acid sequence which includes substitution, deletion, insertion or addition of 1 to 20 amino acids in SEQ ID NO.: 2.

3. Method according to claim 1 or according to claim 2, **characterized in that** in the L-tryptophan producing microorganism, the expression level of the mdfA gene or of the mdfA allele is enhanced by increasing the copy number of the mdfA gene or of the mdfA allele by at least 1.

4. Method according to anyone of the preceding claims, the method being **characterized in that** in the L-tryptophan producing microorganism, the copy number of the mdfA gene or of the mdfA allele is increased by at least 1 by integrating the gene into the chromosome of the microorganism.

5. Method according to anyone of the preceding claims, the method being **characterized in that** in the L-tryptophan producing microorganism, the expression level of the mdfA gene or of the mdfA allele is enhanced by modifying the expression regulatory sequence of the gene.

6. Method according to any one of the preceding claims, the method being **characterized in that** the expression level of the mdfA gene or of the mdfA allele is enhanced by using an inducible promoter.

7. Method according to any one of the preceding claims, the method being **characterized in that** a copy of the mdfA gene or of the mdfA allele is integrated in the mtr locus with simultaneous deletion of the mtr gene.

8. Method according to claim 7, the method being **characterized in that** the chromosomal environment of the mtr locus regulates the expression of the mdfA gene or the mdfA allele integrated in the mtr locus.

9. Method according to any one of the preceding claims, the method being **characterized in that** the L-tryptophan producing microorganism is selected from the genera *Escherichia, Erwina* and *Providencia.*

10. Method according to any one of the preceding claims, the method being **characterized in that** the L-tryptophan producing microorganism is *Escherichia coli.*

11. Method for producing a L-tryptophan producing microorganism by transformation, transduction or conjugation, the method being **characterized in that** the transformation, transduction or conjugation is done using a vector comprising
a) a polynucleotide sequence comprising the nucleotide sequence of SEQ ID NO.: 1;
b) a polynucleotide that hybridizes with a complementary strand of SEQ ID NO.: 1 under stringent conditions;
c) a naturally occurring mutant or polymorphic form of a polynucleotide according to a) or b);
d) a polynucleotide having a sequence identity of at least 80% of the nucleotide sequence of SEQ ID NO.: 1;
e) a polynucleotide including substitutions, deletions, insertions or additions of 1 to 60 nucleotides in relation to the nucleotide sequence of SEQ ID NO.:1;
f) a polynucleotide encoding a protein comprising the amino acid sequence of SEQ ID NO.: 2;
g) a polynucleotide encoding a protein comprising an amino acid sequence which includes substitution, deletion, insertion or addition of 1 to 20 amino acids in SEQ ID NO.: 2;
and comprising a promoter regulating the expression of polynucleotides a) to g).

12. L-tryptophan producing microorganism of the *Enterobacteriaceae* family, the microorganism being **characterized in that** the microorganism has been modified by enhancing the expression level of the mdfA gene or or by enhancing the expression level of an mdfA allele.

13. L-tryptophan producing microorganism according to claim 12, **characterized in that** the mdfA allele is a polynucleotide selected from the group consisting of:
a) a polynucleotide sequence comprising the nucleotide sequence of SEQ ID NO.: 1;
b) a polynucleotide that hybridizes with a complementary strand of SEQ ID NO.: 1 under stringent conditions;
c) a naturally occurring mutant or polymorphic form of a polynucleotide according to a) or b);
d) a polynucleotide having a sequence identity of at least 80% of the nucleotide sequence of SEQ ID NO.: 1;
e) a polynucleotide including substitutions, deletions, insertions or additions of 1 to 60 nucleotides in relation to the nucleotide sequence of SEQ ID NO.: 1;
f) a polynucleotide encoding a protein comprising the amino acid sequence of SEQ ID NO.: 2;
g) a polynucleotide encoding a protein comprising an amino acid sequence which includes substitution, deletion, insertion or addition of 1 to 20 amino acids in SEQ ID NO.: 2.

14. L-tryptophan producing microorganism according to claim 12 or according to claim 13, **characterized in that** a copy of the mdfA gene or of the mdfA allele is integrated in the mtr locus with simultaneous deletion of the mtr gene, and optionally **in that** the chromosomal environment of the mtr locus regulates the expression of the mdfA gene or the mdfA allele integrated in the mtr locus.

15. Use of the L-tryptophan producing microorganism according to any one of claims 12 to 14 for producing L- tryptophan or, alternatively, for producing a feedstuff additive containing L-tryptophan.
